(19) Europäisches Patentamt European Patent Office Office européen des brevets

(11) **EP 3 181 699 A1**

(12) **EUROPEAN PATENT APPLICATION**

(43) Date of publication:
**21.06.2017  Bulletin 2017/25**

(51) Int Cl.:
**C12Q 1/68** (2006.01)

(21) Application number: **15200884.3**

(22) Date of filing: **17.12.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **ONIRIS**
**44307 Nantes Cedex 3 (FR)**
• **Université de Nantes**
**44035 Nantes (FR)**

(72) Inventors:
• **BACH, Jean-Marie**
**44470 CARQUEFOU (FR)**
• **BOSCH, Steffi**
**44470 MAUVES SUR LOIRE (FR)**
• **SALAMA, Apolline**
**44100 NANTES (FR)**

(74) Representative: **Cabinet Plasseraud**
**66 rue de la Chaussée d'Antin**
**75440 Paris Cedex 09 (FR)**

(54) **MICRORNAS AS PREDICTIVE BIOMARKERS OF TYPE 1 DIABETES**

(57)     The present invention relates to microRNAs (miR-7a, miR-215 and miR-375) for use in the *in vitro* diagnosis of insulitis and the *in vitro* prognosis of future type 1 diabetes development. The invention also relates to miR-specific primers and probes, kits comprising these primers and/or probes, and *in vitro* methods of diagnosis and prognosis using these primers and/or probes. Also provided are methods for assessing the effectiveness of a therapeutic treatment for insulitis or of a treatment for preventing type 1 diabetes onset, as well as methods of screening for candidate compounds potentially useful in the treatment for insulitis or in the prevention of type 1 diabetes onset.

Figure 2

**SEQUENCE LISTING**

```
<110>  SATT OUEST
       BACH , Jean-Marie et al.

<120>  MicroRNAs as Predictive Biomarkers of Type 1 Diabetes

<130>  BEP151015EP

<160>  3

<170>  PatentIn version 3.5

<210>  1
<211>  23
<212>  RNA
<213>  Homo sapiens

<400>  1
uggaagacua gugauuuugu ugu                                          23


<210>  2
<211>  21
<212>  RNA
<213>  Homo sapiens

<400>  2
augaccuaug aauugacaga c                                            21


<210>  3
<211>  22
<212>  RNA
<213>  Homo sapiens

<400>  3
uuuguucguu cggcucgcgu ga                                           22
```

## Description

### Background of the Invention

Type 1 diabetes (also known as type 1 diabetes mellitus), which generally develops in children, is a serious chronic disease with an unknown cause. It is characterized by the autoimmune destruction of insulin-producing (beta) β-cellos in the pancreas. The subsequent lack of insulin leads to increased blood and urine glucose. Globally, type 1 diabetes affects between 15 and 30 million people worldwide (World Health Organization). The incidence of childhood onset diabetes is increasing in many countries (Patterson et al., Diabetes Res. Clin. Pract., 2014, 103: 161-175; Tamayo et al., Diabetes Res. Clin. Pract., 2014, 103: 206-217), with an estimated 80,000 children developing the disease each year. Insulin therapy, which is essential for survival of type 1 diabetes patients, must be continued indefinitely and requires multiple daily injections. In addition to insulin therapy, dietary management is important. Untreated or poorly managed diabetes can cause many complications, including serious long-term complications, such as heart disease, stroke, kidney failure, foot ulcers, damage to the eyes, and coma. Complications may also arise from low blood sugar caused by excessive treatment. Although the incidence peak of type 1 diabetes peaks between the ages of 4 and 10, 5% to 10% of adults diagnosed with type 2 diabetes are believed to suffer from slow onset type 1 diabetes (also known as latent autoimmune diabetes of adults or LADA), which is characterized by a very long pre-diabetic period.

Type 1 diabetes is clinically diagnosed when about 85% of the initial stock of pancreatic insulin-producing β-cells are destroyed. In humans, as well as in non-obese diabetic (NOD) mice (an animal model of spontaneous type 1 diabetes), the onset of the disease occurs after an asymptomatic period called prediabetes, during which the autoimmune reaction has started, but the functional β-cells are sufficiently numerous to maintain glucose homeostasis. The prediabetic period could be made use of for an immune-intervention with the goal of preserving pancreatic β-cells and preventing type 1 diabetes development.

The diagnostic methods currently used (dosage of auto-antibodies, and genotyping of alleles of the HLA (human leucocyte antigen) system) do not allow to efficiently and precisely determine if and when (during childhood, teenage years or adulthood) a prediabetic patient will develop type 1 diabetes. Indeed, the detection of auto-antibodies (such as auto-antibodies directed against glutamic acid decarboxylase (GAD65), tyrosine phosphatase-like protein (IA-2) or insulin (IAA)), which are present during the prediabetic period, provides limited information in terms of autoimmunity evolution and future clinical development of type 1 diabetes (Ziegler and Nepom, Immunity, 2010, 32: 468-478; Watkins et al., Transl. Res., 2014, 164: 110-121). Similarly, genetic factors do not solely explain the onset of the autoimmune process and have no predictive value for the subsequent development of the disease.

Most studies aimed at identifying prediabetic patients or patients at risk of developing type 1 diabetes are performed on prediabetic patients with first degree relatives diagnosed with the disease. It was found that children with a family background of type 1 diabetes, who test positive for at least 3 auto-antibodies out of 5 tested, and who exhibit associated metabolic parameters have a high risk (about 90%) of developing type 1 diabetes within three years (Sosenko et al., Diabetes Care, 2014, 37: 979-984). However, only 10% of type 1 diabetics have first-degree relatives diagnosed with the disease.

Retrospective studies of the general population have shown that all type 1 diabetes patients have auto-antibodies before the onset of the disease. When multiple auto-antibodies were tested, up to 100% of type 1 diabetes patients were found to be positive before diagnosis, although there was no correlation between auto-antibodies positivity and length of prediabetes period (Knip et al., Diabetes Care, 2010, 33: 1206-1212). Thus, auto-antibodies can provide highly sensitive tests in the general population. However, the low risk of type 1 diabetes development in the general population (about 0.4%) is associated with a high rate of false positives. Only 50% of children or teenagers of the general population, who test positive for one or several auto-antibodies, develop the disease in the following years (Landin-Olsson et al., Diabetologia, 1992, 35: 1068-1073; La Gasse et al., Diabetes Care, 2002, 26: 505-511; Knip et al., Diabetes Care, 2010, 33: 1206-1212), indicative of the low predictive value and specificity of auto-antibodies tests. These tests are also limited by fluctuations of auto-antibody titers over time (Karjalainen, Diabetes, 1990, 39: 1144-1150; La Gasse et al., Diabetes Care, 2002, 26: 505-511).

Micro-RNAs (miRNAs) are small non-coding RNA molecules, which in recent years have emerged to have enormous potential as biomarkers. Several micro-RNA biomarkers of type 1 diabetes have been identified (see, for example, Latreille et al., J. Clin. Invest., 2014, 124: 2722; Mu et al., PloS One, 2013, 8:e58622; Nielsen et al., Exp. Diabetes Res., 2012, doi:896362; Salama et al., PloS One, 2004, 9: e106153; Sebastiani et al., Diabetes Metab. Res. Rev., 2011, 27: 862; WO 2009/067644; EP 2 419 536; WO 2012/101392; WO2014/022852; and EP 2 545 190). Erener et al. (Endocrinology, 2013, 154: 603-608) have shown that in the NOD mouse model of autoimmune diabetes, circulating miR-375 levels are significantly increased 2 weeks before diabetes onset. However, this short delay does not allow any practical application in the human clinical field.

Thus, there still remains, in the art, an ongoing need for new strategies for the early diagnostic of type 1 prediabetes in the general population and the development of reliable prognostic tools for identifying asymptomatic patients with a high risk for progression to type 1 diabetes.

## Summary of the Invention

Using the NOD mouse model, the present Inventors have observed that (1) the expression of serum miR-7a correlates with the degree of insulitis in young NOD mice exhibiting early insulitis; (2) the variations of expressions levels of miR-7a, miR-215 and miR-375, taken together, correlate with the degree of insulitis in prediabetic NOD mice, and (3) the serum level of miR-7a is significantly increased during at least 3 months before onset of type 1 diabetes, and is associated with future development of type 1 diabetes. This offers a larger time frame for therapeutic intervention.

Accordingly, in one aspect, the present invention provides an *in vitro* method for predicting future type 1 diabetes development in a subject, the method comprising a step of: determining the level of miR-7a in a biological sample obtained from said subject.

In certain embodiments, the subject is a type 1 prediabetic subject.

In certain embodiments, the biological sample used in the *in vitro* method is a blood sample selected from the group consisting of a whole blood sample, a serum sample and a plasma sample or the biological sample comprises lipoproteins (such as HDLs; LDLs...), extracellular vesicles (such as exosomes, microvesicles, apoptotic bodies...) or Argonaute protein complexes extracted from a blood sample taken from said subject.

In certain embodiments, the *in vitro* method further comprises a step of: comparing the level of miR-7a measured in the biological sample obtained from the subject to the level of miR-7a measured in a biological sample obtained from at least one healthy subject, wherein an increased miR-7a level in the tested subject compared to the healthy subject is indicative of future development of type 1 diabetes in the tested subject.

Determining the level of miR-7a in the biological sample may be performed using any suitable method known in the art. In certain embodiments, the level of miR-7a in the biological sample is performed using a polymerase chain reaction (PCR) technique.

The present invention also provides an *in vitro* method for assessing the effectiveness of a therapeutic treatment administered to a type 1 prediabetic subject for preventing future type 1 diabetes development in said prediabetic subject, the method comprising steps of: (a) determining the level of miR-7a in a biological sample obtained from the type 1 prediabetic subject after administration of the therapeutic treatment; and (b) comparing the level of miR-7a measured in step (a) to the level of miR-7a measured in a biological sample obtained from the type 1 prediabetic subject before administration of the therapeutic treatment. A decreased miR-7 level after treatment compared to before treatment is indicative of a therapeutic treatment that is effective/efficient in said type 1 prediabetic subject.

The present invention further provides a method for screening candidate compounds for their ability to prevent future type 1 diabetes development in prediabetic subjects, said method comprising steps of: (a) determining the level of miR-7a in a cell after contacting the cell with the candidate compound or determining the level of miR-7a in a biological sample obtained from an animal model after administration of the candidate compound to the animal model; and (b) comparing the level of miR-7a measured in step (a) to the level of miR-7a measured in a cell before contacting the cell with the candidate compound or to the level of miR-7a measured in a biological sample obtained from the animal model before administering the candidate compound to the animal model, wherein a decreased miR-7 level after contacting/administration compared to before contacting/administration is indicative of an efficient candidate compound, i.e., a candidate compound that is potentially useful for preventing future type 1 diabetes development in prediabetic subjects.

The present invention also provides an *in vitro* method for diagnosing early insulitis in a subject, the method comprising steps of: (a) determining the level of miR-7a in a biological sample obtained from said subject; (b) comparing the level of miR-7a measured in step (a) to the level of miR-7a measured in a biological sample obtained from at least one healthy subject, wherein an increased miR-7a level in the tested subject compared to the healthy subject is indicative of early insulitis in the tested subject.

The present invention also provides an *in vitro* method for staging insulitis in a subject, the method comprising steps of: (a) determining the levels of miR-7a, miR-215 and miR-375 in a biological sample obtained from said subject; and (b) combining the levels of miR-7a, miR-215 and miR-375 to obtain the stage/score of insulitis in said subject.

In certain embodiments, the step of combining the levels of miR-7a, miR-215 and miR-375 to obtain the stage/score of insulitis in said subject comprises calculating the stage/score of insulitis (IS) as:

$$IS = 0.65 + 0.25 \, RQ(miR\text{-}7a) + 0.66 \, RQ(miR\text{-}215) - 0.42 \, RQ(miR\text{-}375),$$

wherein RQ(miR-7a), RQ(miR-215) and RQ(miR-375) are the levels of the miR-7a, miR-215 and miR-375, respectively measured in the biological sample.

In other embodiments, the combining the levels of miR-7a, miR-215 and miR-375 to obtain the stage/score of insulitis in said subject comprises calculating the stage/score of insulitis (IS) as:

$$IS = 0.6 + 0.2\ RQ(miR\text{-}7a) + 0.6\ RQ(miR\text{-}215) - 0.4\ RQ(miR\text{-}375),$$

wherein RQ(miR-7a), RQ(miR-215) and RQ(miR-375) are the levels of the miR-7a, miR-215 and miR-375, respectively measured in the biological sample.

The present invention also provides an *in vitro* method for diagnosis insulitis in a subject, the method comprising steps of: (a) determining the levels of miR-7a, miR-215 and miR-375 in a biological sample obtained from said subject; (b) combining the levels of miR-7a, miR-215 and miR-375 to obtain a combined value for the subject; and (c) comparing the combined value obtained in (b) with the combined value obtained by combining the levels of miR-7a, miR-215 and miR-375 measured in a biological sample obtained from at least one healthy subject, wherein an increased combined value in the tested subject compared to the healthy subject is indicative of insulitis in the tested subject.

In certain embodiments, combining the levels of miR-7a, miR-215 and miR-375 to obtain a combined value comprises calculating the combined value (CV) as:

$$CV = 0.25\ RQ(miR\text{-}7a) + 0.66\ RQ(miR\text{-}215) - 0.42\ RQ(miR\text{-}375),$$

wherein RQ(miR-7a), RQ(miR-215) and RQ(miR-375) are the levels of the miR-7a, miR-215 and miR-375, respectively measured in the biological sample.

In other embodiments, combining the levels of miR-7a, miR-215 and miR-375 to obtain a combined value comprises calculating the combined value (CV) as:

$$CV = 0.2\ RQ(miR\text{-}7a) + 0.6\ RQ(miR\text{-}215) - 0.4\ RQ(miR\text{-}375),$$

wherein RQ(miR-7a), RQ(miR-215) and RQ(miR-375) are the levels of the miR-7a, miR-215 and miR-375, respectively measured in the biological sample.

The present invention also provides an *in vitro* method for assessing the effectiveness of a therapeutic treatment administered to a subject to treat insulitis, the method comprising steps of: (a) determining the levels of miR-7a, miR-215 and miR-375 in a biological sample obtained from said subject after administration of the therapeutic treatment; (b) combining the levels of miR-7a, miR-215 and miR-375 to obtain a combined value after administration; and (c) comparing the combined value obtained in (b) with the combined value obtained by combining the levels of miR-7a, miR-215 and miR-375 measured in a biological sample obtained from at said subject before administration of the therapeutic treatment, wherein a decreased combined value after treatment compared to before treatment is indicative of the effectiveness of the therapeutic treatment in said subject.

The combined values may be calculated as described above.

In a second aspect, the present invention provides a miR-7a primer and/or probe for use in an *in vitro* method of predicting future type 1 diabetes development in a subject, or for use in an *in vitro* method of diagnosing early insulitis.

The present invention also provides a set of primers and/or probes for use in an *in vitro* method of diagnosing insulitis in a subject, wherein the set comprises at least one miR-7a primer and/or probe, at least one miR-215 primer and/or probe, and at least one miR-375 primer and/or probe.

In a third aspect, the present invention provides a kit for use in an *in vitro* method of predicting future type 1 diabetes development in a subject, or for use in an *in vitro* method of diagnosing early insulitis, said kit comprising a miR-7a primer and/or probe and instructions to perform a diagnosis according to a method described herein.

The present invention also provides a kit for use in an *in vitro* method of diagnosing or staging insulitis in a subject, said kit comprising at least one miR-7a primer and/or probe, at least one miR-215 primer and/or probe, and at least one miR-375 primer and/or probe, and instructions to perform insulitis staging or insulitis diagnosis according to a method described herein.

These and other objects, advantages and features of the present invention will become apparent to those of ordinary skill in the art having read the following detailed description of the preferred embodiments.

**Definitions**

Throughout the description, several terms are employed that are defined in the following paragraphs.

As used herein, the term *"subject"* refers to a human or another mammal (*e.g.*, primate, dog, cat, goat, horse, pig, mouse, rat, rabbit, and the like) that can develop type 1 diabetes, but may or may not have the disease. Non-human subjects may be transgenic or otherwise modified animals. In many embodiments of the present invention, the subject is a human being. In such embodiments, the subject is often referred to as an *"individual"* or a "*patient*". The terms "subject", "individual" and "patient" do not denote a particular age, and thus encompass newborns, children, teenagers, and adults. The term "patient" more specifically refers to an individual suffering from a disease (*e.g.*, type 1 diabetes) or diagnosed with a clinical condition (*e.g.*, type 1 prediabetes).

The term **"*type 1 prediabetic subject*"**, as used herein, refers to a subject that has been diagnosed with type 1 prediabetes. The diagnostic may have been performed using any method known in the art, in particular methods that are currently in clinical use for diagnosing type 1 prediabetes, and/or using a method according to the present invention.

The terms "*biomarker*" and "*marker*" are used herein interchangeably. They refer to a substance that is a distinctive indicator of a biological process, biological event and/or pathological condition. In the context of the present invention, a high level of the micro-RNA, miR-7a, has been identified, by the present Inventors, to be a predictive marker of insulitis and of future type 1 diabetes development. The phrase "*predictive marker of future type 1 diabetes development*" is used herein to refer to a biological process or event (here: high levels of miR-7a) that is prognostic of future development of type 1 diabetes, in that the biological process or event is found significantly more often in a subject that is going to develop type 1 diabetes than in a subject that is not going to develop type 1 diabetes (as determined using routine statistical methods). Similarly, the present Inventors have found that high levels of both miR-7a and miR-215 associated with a low level of miR-375 are correlated with a high degree of insulitis, while a high level of miR-7a is indicative of early insulitis. As used, herein, the term "*insulitis*" has its art-understood meaning, and refers to an inflammation of the islets of Langerhans of the pancreas. The endocrine pancreas becomes infiltrated by polymorphonuclear leukocytes and mononuclear cells, leading to inflammation. The term "*early insulitis*" more particularly refers to a phase of insulitis associated with a low degree of infiltration by polymorphonuclear leukocytes and mononuclear cells.

The term "*micro-RNA*" (or "*miRNA*"). as used herein, has its art-understood meaning. Micro-RNAs are a major group of noncoding RNAs that are known to regulate almost a third of all the coding genes. They are small (~ 20-25 nucleotides long) endogenously formed repressors of gene expression. Micro-RNAs usually bind to the 3' untranslated region (3'UTR) of the target RNA transcripts (mRNAs or circRNAs) and are capable of inducing posttranscriptional gene regulation by blocking translation or by degrading the target RNAs, or by doing both.

The term *"biological sample"* is used herein in its broadest sense. A biological sample is generally obtained from a subject. A biological sample may be any biological tissue or fluid with which a biomarker of the present invention may be assayed. The term also includes archival samples with known diagnosis, treatment and/or outcome history. Examples of biological samples include, but are not limited to, blood samples. The term "*blood sample*" refers to a sample of blood taken from a subject. The blood sample may comprise arterial, capillary and/or venous blood. The blood sample may be used as a whole blood sample, or may be separated to yield plasma and/or serum. The term "biological sample" also encompasses any material derived by processing a biological sample. Derived materials include, but are not limited to, cells (or their progeny) isolated from the sample, as well as extracellular vesicles (*e.g.,* exosomes, microvesicles, apoptotic bodies...), lipoproteins (*e.g.,* high-density lipoprotein (HDL), low-density lipoprotein (LDL), Argonaute protein complexes, or molecules (*e.g.*, total RNA including micro-RNAs) extracted from the sample. Processing of a biological sample may involve one or more of: filtration, distillation, extraction, concentration, inactivation of interfering components, addition of reagents, and the like. In certain embodiments of the invention, the biological sample comprises lipoproteins such as HDLs and/or LDLs. As used herein, the terms *"HDLs"* and *"LDLs"* have their art understood meanings and refer to complex particles composed of multiple proteins which transport lipids (*e.g.*, cholesterol, phospholipids and triglycerides) around the body within the extracellular fluid. In certain embodiments of the invention, the biological sample comprises exosomes. As used herein, the term *"exosomes"* has its art-understood meaning and refers to cell-derived vesicles that are present in many and perhaps all biological fluids, including blood, urine, and culture medium of cell cultures. The reported diameter of exosomes is between 30 and 100 nm, which is larger than LDL, but much smaller than red blood cells.

In a diagnostic method according to the present invention, the biological sample is obtained from the subject to be tested and the results of the test are compared to those obtained for a biological sample of a control subject. The biological samples obtained from the subject to be tested and from the control subject are preferably similarly processed. As used herein, the term *"similarly processed"* refers to samples, which have been obtained and/or manipulated using the same protocol. In the context of the present invention, the term *"control"*, when used herein to characterize a subject, refers to a subject that is healthy or to a patient with a known diagnosis and/or outcome history. In certain embodiments, control subjects have the same age, sex, and/or body mass index as compared to the subject to be tested. The terms "*normal*" and "*healthy*" are used herein interchangeably. When used herein to characterize a subject, they refer to a subject that does not suffer from type 1 prediabetes or type 1 diabetes. The terms "normal" and "healthy" are also used herein to qualify a biological sample obtained from a healthy subject (or from a cohort or population of healthy subjects). A patient with a known diagnosis and/or outcome history may be a patient diagnosed with type 1 prediabetes, or a patient diagnosed with type 1 prediabetes but who has not developed type 1 diabetes, or yet a patient diagnosed with type 1 diabetes. The term *"control sample"* refers to one or more than one sample that has been obtained from a control subject (or from a cohort or population of control subjects). In some embodiments, the "control sample" is a pre-determined value, threshold or range.

The term *"oligonucleotide"*, as used herein, refers to a short string of a deoxyribonucleotide or ribonucleotide polymer. Oligonucleotides can be used as amplification primers and/or detection probes and/or spikes for technical internal control. Such short stretches of nucleic acid sequences are often chemically synthesized. As will be appreciated by those skilled in the art, the length of an oligonucleotide can vary widely, often depending on its intended function or use. Generally, oligonucleotides comprise between about 5 and about 150 nucleotides, preferably between about 15 and about 100 nucleotides, more preferably between about 15 and about 50 nucleotides.

The term "hybridization" refers to the formation of complexes between nucleotide sequences, which are sufficiently complementary to form complexes *via* Watson-Crick base pairing or non-canonical base pairing. When a primer "hybridizes" with a target sequence (template), such complexes (or hybrids) are sufficiently stable to serve the priming function required by, *e.g.,* the DNA polymerase, to initiate DNA synthesis. It will be appreciated that hybridizing sequences need not have perfect complementarity to provide stable hybrids. In many situations, stable hybrids will form where fewer than about 10% of the bases are mismatches. Those skilled in the art understand how to estimate and adjust the stringency of hybridization conditions such that sequences having at least a desired level of complementarity will stably hybridize, while those having lower complementarity will not. For examples of hybridization conditions and parameters see, *e.g.,* J. Sambrook et al., "Molecular Cloning: A Laboratory Manual", 2012, 4th Ed., Cold Spring Harbor Press: Plainview, NY; F.M. Ausubel, "Current Protocols irt Molecular Biology", 1994, John Wiley & Sons: Secaucus, NJ. As used herein, the term "*specifically hybridizes*" means that cross-hybridization with micro-RNAs other than a micro-RNA biomarker of the invention is not significantly produced under ordinary hybridization conditions, preferably under stringent hybridization conditions (see, for example, the conditions disclosed in Sambrook et al., "Molecular Cloning: A Laboratory Manual", 2012, 4th Ed., Cold Spring Harbor Press: Plainview, NY).

As used herein, the term "*amplification*" refers to a method or process that increases the representation of a population of specific nucleic acid sequences in a sample. Amplification methods (such as polymerase chain reaction or reverse transcription polymerase chain reaction) are known in the art.

The terms "*target sequence*" and "*target nucleic acid*" are used herein interchangeably. They refer to a nucleic acid sequence, the presence or absence of which is desired to be detected. In the context of the present invention, the target sequence preferably includes a nucleic acid sequence to which oligonucleotide primers will hybridize. The target sequence may also include a probe-hybridizing region with which a probe will form a stable hybrid under desired conditions.

The terms *"primer"* and "*amplification primer*" are used herein interchangeably. They refer to an isolated oligonucleotide, which is capable of acting as a point of initiation of synthesis of a primer extension product, when placed under suitable conditions (*e.g.*, buffer, salt, temperature and pH) in the presence of nucleotides and an agent for nucleic acid polymerization (*e.g.*, a DNA-dependent or RNA-dependent polymerase). The primer is preferably single-stranded for maximum efficiency in amplification, but may alternatively be double-stranded, in particular partially double-stranded or stem-loop primer. A typical amplification primer contains about 10 to about 50 nucleotides in length of a sequence substantially complementary to the target sequence. However, a primer can also contain additional sequences. For example, amplification primers used in Strand Displacement Amplification (SDA) preferably include a restriction endonuclease recognition at site 5' to the target binding sequence (see, for example, U.S. Pat. Nos 5,270,184 and 5,455,166). Nucleic Acid Sequence Based Amplification (NASBA), and Transcription-Mediated Amplification (TMA) primers preferably include an RNA polymerase promoter linked to the target binding sequence of the primer. Methods for linking such specialized sequences to a binding target sequence for use in a selected amplification reaction are well-known in the art.

The term *"amplification conditions"*, as used herein, refers to conditions that promote annealing and/or extension of primer sequences. Such conditions are well-known in the art and depend on the amplification method selected. Thus, for example, in a PCR reaction, amplification conditions generally comprise thermal cycling, *i.e.,* cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, pH and the like.

As used herein, the term *"amplification reaction reagents"* refers to reagents used in nucleic acid amplification reactions and may include, but are not limited to, buffers, reagents, enzymes having reverse transcriptase and/or polymerase activity or exonuclease activity; enzyme cofactors such as magnesium or manganese; salts; and deoxynucleotide triphosphates (dNTPs) such as deoxyadenosine triphosphate (dATP), deoxyguanosine triphosphate (dGTP), deoxycytidine triphosphate (dCTP), deoxythymidine triphosphate (dTTP) and deoxyuridine triphosphate (dUTP). Amplification reaction reagents may readily be selected by one skilled in the art depending on the amplification method used.

The terms *"probe"* and *"detection probe"* are used herein interchangeably and refer to an oligonucleotide capable of selectively hybridizing to at least a portion of a target sequence under appropriate conditions (*e.g.*, a portion of a target sequence that has been amplified). In general, a probe sequence is identified as being either "complementary" (*i.e.,* complementary to the coding or sense strand (+)), or "reverse complementary" (*i.e.,* complementary to the anti-sense strand (-)). In certain embodiments, a detection probe is labeled with a detectable agent or moiety.

The terms *"labeled", "labeled with a detectable agent"* and *"labeled with a detectable moiety"* are used herein interchangeably. These terms are used to specify that an entity (*e.g.,* a probe) can be visualized, for example, following binding to another entity (*e.g.,* a target sequence). Preferably, a detectable agent or moiety is selected such that it generates a signal which can be measured and whose intensity is related to the amount of bound entity. In array-based methods, a detectable agent or moiety is also preferably selected such that it generates a localized signal, thereby allowing spatial resolution of the signal from each spot on the array. Methods for labeling probes are well known in the art. Labeled probes can be prepared by incorporation of or conjugation (*e.g.,* through a covalent bond) to a label, that is directly or indirectly detectable by spectroscopic, photochemical, biochemical, immunochemical, electrical, optical, or chemical means, or any suitable means. Suitable detectable agents include, but are not limited to, various ligands, radionuclides, fluorescent dyes, chemiluminescent agents, microparticles, enzymes, colorimetric labels, magnetic labels, and haptens.

The term *"directly **detectable"*,* when used herein in reference to a label or detectable moiety, means that the label or detectable moiety does not require further reaction or manipulation to be detectable. For example, a fluorescent moiety is directly detectable by fluorescence spectroscopy methods. The term *"indirectly detectable"*, when used herein in reference to a label or detectable moiety, means that the label or detectable moiety becomes detectable after further reaction or manipulation. For example, a hapten becomes detectable after reaction with an appropriate antibody attached to a reporter, such as a fluorescent dye.

The term "*isolated*" when referring herein to an oligonucleotide or to a micro-RNA means an oligonucleotide or micro-RNA, which, by virtue of its origin or manipulation, is separated from at least some of the components with which it is naturally associated or with which it is associated when initially obtained or prepared. By "isolated", it is alternatively or additionally meant that the oligonucleotide, is produced or synthesized by the hand of man.

The term *"treatment"* is used herein to characterize a method or process that is aimed at (1) delaying or preventing the onset of a disease or condition (here type 1 diabetes); (2) slowing down or stopping the progression, aggravation, or deterioration of the symptoms of the disease or condition; (3) bringing about amelioration of the symptoms of the disease or condition; or (4) curing the disease or condition. A treatment may be administered after initiation of the disease or condition, for a therapeutic action. Alternatively, a treatment may be administered prior to the onset of the disease or condition, for a prophylactic or preventive action. In this case, the term "*prevention*" is used.

The terms *"approximately"* and *"about",* as used herein in reference to a number, generally include numbers that fall within a range of 10% in either direction of the number (greater than or less than the number) unless otherwise stated or otherwise evident from the context (except where such number would exceed 100% of a possible value).

## Detailed Description of Certain Preferred Embodiments

As mentioned above, miR-7a is described herein as a predictive marker of future type 1 diabetes development and as an indicator of early insulitis; and a set of the three micro-RNAs, miR-7a, miR-215 and miR-375, is described as a diagnostic marker of insulitis. These biomarkers allow the identification of early type 1 prediabetics and of individuals with high risk of developing type 1 diabetes in the future. Therefore, this enables preventive action to be taken, in particular medical intervention, to preserve functioning pancreatic beta cells and prevent type 1 development. Furthermore, the biomarkers can be used to assess the effectiveness of a treatment or to screen potential therapeutic candidate compounds.

## I - micro-RNA Biomarkers

MicroRNAs (or miRNAs) are a class of small non-coding RNAs that function as translational repressors. They bind through canonical base pairing to a complementary site in the 3' untranslated region (UTR) of their target mRNAs and can direct degradation or translational repression of these transcripts. They have been shown to play important roles in development, stress responses, angiogenesis and oncogenesis. Once thought to be unstable RNA molecules, micro-RNAs are now known to be stably expressed in serum, plasma, urine, saliva, and other body fluids, wherein they are not cell-associated, but transported with in lipoproteins (*e.g.,* HDLs or LDLs), Argonaute protein complexes or packaged in extracellular vesicles (*e.g.,* exosomes, microvesicles, apoptotic bodies) that protect them from endogenous RNase activity (Mitchell et al., Proc. Natl. Acad. Sci. USA, 2008, 105: 10513-10518). The presence of these circulating micro-RNAs in bodily fluids makes them particularly useful as non-invasive biomarkers.

The present Inventors have identified three circulating micro-RNAs, miR-7a, miR-215 and miR-375, that are useful as diagnostic biomarkers of type 1 prediabetes, and in particular as diagnostic biomarkers of insulitis, and as predictive biomarkers of future development of type 1 diabetes.

As used herein, the term *"miR-7a"* refers to an evolutionarily conserved miRNA (see miRBase Family Number: MIPF0000022). In humans, miR-7a is encoded by three different genomic loci. Thus, human miR-7a is derived from three stem-loop (or hairpin) microRNA precursors in the genome: miR-7-1 (whose sequence is given by miRBase Number: MI0000263, and which is encoded by the *MIR7-1* gene located on the long (q) arm of chromosome 9 at location 21.32), miR-7-2 (whose sequence is given by miRBase Number: MI0000264, and which is encoded by the *MIR7-2* gene located on the long (q) arm of chromosome 15 at location 26.1) and miR-7-3 (whose sequence is given by miRBase Number: MI0000265, and which is encoded by the *MIR7-3* gene located on the (p) arm of chromosome 19 at location 13.3).

More specifically, the term "miR-7a" (also called miR7-5p) refers to the mature sequence of human miR-7a, which is given by miRBase Number: MIMAT0000252. This sequence is:

SEQ ID NO: 1: uggaagacuagugauuuuguugu.

The sequence of human miR-7a is identical or highly similar to the mature sequence of miR-7a of other mammal species including the horse (miRBase Number: MIMAT0012905), rat (miRBase Number: MIMAT0000606), bovine (miRBase Number: MIMAT0003843), mouse (miRBase Number: MIMAT0000677), dog (miRBase Number: MIMAT0006634), rhesus macaque (miRBase Number: MIMAT0006159), goat (miRBase Number: MIMAT0036288), hamster (miRBase Number: MIMAT0020368), wild boar (miRBase Number: MIMAT0002141), chimpanzee (miRBase Number: MIMAT0002485), etc...

As used herein the term *"miR-215"* refers to a micro-RNA that is homologous to miR-192 and that, in humans, is encoded by the *MIR215* gene, which is located on the long (q) arm of chromosome 1 at location 41. The stem loop sequence of miR-215 is given by miRBase Number: MI0000291. The mature sequence of miR215-5p is given by miRBase Number: MIMAT0000272 and the mature sequence of miR215-3p is given by miRBase Number: MIMAT0026476.

In the context of the present invention, the term "miR-215" refers to the mature sequence of human miR215-5p, which is set forth in SEQ ID NO: 2:

SEQ ID NO 2: augaccuaugaauugacagac.

The sequence of miR-215 is known for other species (see miRBase Family Number: MIPF0000063, which lists 48 sequences). The mature sequence of human miR-215 is identical or highly similar to the mature sequence of miR-215 of other mammal species including the horse (miRBase Number: MIMAT0013190), bovine (miRBase Number: MIMAT0003797), rhesus macaque (miRBase Number: MIMAT0002728), goat (miRBase Number: MIMAT0036060), hamster (miRBase Number: MIMAT0023853), wild boar (miRBase Number: MIMAT0010192), chimpanzee (miRBase Number: MIMAT0002730), orangutan (miRBase Number: MIMAT0002732), gorilla (MIMAT0002734), pigtail macaque, (MIMAT0002736), opossum (MIMAT0012751), etc...

As used herein the term *"miR-375"* refers to a micro-RNA that, in humans, is encoded by the *MIR375* gene, which is located on the long (q) arm of chromosome 2 at location 35. In humans, the mature sequence of miR-375 is given by miRBase Number: MIMAT0000728, and the stem loop sequence of miR-375 is given by miRBase Number: MI0000783. The term "miR-375" more specifically refers to the mature sequence of human miR-375, which has the following sequence:

SEQ ID NO: 3: uuuguucguucggcucgcguga.

The sequence of miR-375 is known for other species (see miRBase Family Number: MIPF0000114, which lists 49 sequences). The mature sequence of human miR-375 is identical or highly similar to the mature sequence of miR-375 of other mammal species including the dog (miRBase Number: MIMAT0009871), bovine (miRBase Number: MIMAT0009303), rat (miRBase Number: MIMAT0005307), mouse (miRBase Number: MIMAT0000739), chimpanzee (miRBase Number: MIMAT0008125), orangutan (miRBase Number: MIMAT0015863), rhesus macaque (miRBase Number: MIMAT0006302), gorilla (miRBase Number: MIMAT0024189), opossum (miRBase Number: MIMAT0004138), etc....

**II - Methods using the micro-RNA Biomarkers**

As will be understood by those of ordinary skill in the art, biomarkers whose expression level correlates with the degree of insulitis and/or with future development of type 1 diabetes, can be used to characterize biological samples obtained from subjects and thereby provide a diagnosis or prognosis regarding these subjects. These biomarkers can also be used in the assessment of the effectiveness of a therapeutic treatment or in the screening of potential therapeutic candidates.

## A. Biological Samples

The methods of the present invention comprise: determining the level of miR-7a or the levels of miR-7a, miR-215 and miR-375 in a biological sample obtained from the subject to be tested. The methods described herein may be applied to the testing of any biological sample allowing a microRNA biomarker of the invention to be assayed. The biological sample is generally obtained from a subject to be tested. The subject is generally a mammal. In certain preferred embodiments of the present invention, the subject to be tested is a human being.

In certain embodiments, a method according to the invention is performed on a blood sample taken from the subject to be tested. The blood sample may be a whole blood sample, a serum sample or a plasma serum. In certain embodiments, the inventive methods are performed on the biological sample without any major manipulation of the sample. However, in other embodiments, the inventive methods are performed after extraction of microRNAs from the blood sample or after isolation of extracellular vesicles (such as exosomes, microvesicles, apoptotic bodies), lipoproteins (*e.g.*, HDLs or LDLs), or Argonaute protein complexes from the blood sample and extraction of miRNAs.

Several techniques have been developed to isolate microRNAs from biological samples. The stability of the stored miRNA in samples has often been questioned. MicroRNAs like other RNAs are rapidly degraded because of the ubiquitous presence of RNases. As known in the art, this makes it necessary to cool samples on ice and use RNase-free equipment whenever working with microRNAs.

Total RNA (including miRNAs) may be extracted from biological samples using RNA extraction methods that are well known in the art (see, for example, J. Sambrook et al., "Molecular Cloning: A Laboratory Manual", 1989, 2nd Ed., Cold Spring Harbour Laboratory Press: New York). Most methods of RNA isolation from bodily fluids or tissues are based on the disruption of the tissue in the presence of protein denaturants to quickly and effectively inactivate RNases. Generally, RNA isolation reagents comprise, among other components, guanidium thiocyanate and/or betamercaptoethanol, which are known to act as RNase inhibitors. Isolated total RNA may then be further purified from the protein contaminants and concentrated by selective ethanol precipitations, phenol/chloroform extractions followed by isopropanol precipitation (see, for example, P. Chomczynski and N. Sacchi, Anal. Biochem., 1987, 162: 156-159) or cesium chloride, lithium chloride or cesium trifluoroacetate gradient centrifugations.

In certain embodiments, after extraction, mRNA is amplified, and transcribed into cDNA, which can then serve as template for multiple rounds of transcription by the appropriate RNA polymerase. Amplification methods are well known in the art (see, for example, A.R. Kimmel and S.L. Berger, Methods Enzymol. 1987, 152: 307-316; J. Sambrook et al., "Molecular Cloning: A Laboratory Martial", 1989, 2nd Ed., Cold Spring Harbour Laboratory Press: New York; "Short Protocols in Molecular Biology", F.M. Ausubel (Ed.), 2002, 5th Ed., John Wiley & Sons; U.S. Pat. Nos. 4,683,195; 4,683,202 and 4,800,159). Reverse transcription reactions may be carried out using non-specific primers, such as an anchored oligo-dT primer, or random sequence primers, or using a target-specific primer complementary to the RNA for each micro-RNA biomarker being monitored (*e.g.*, a micro-RNA specific stem loop reverse-transcription primer), or using thermostable DNA polymerases (such as avian myeloblastosis virus reverse transcriptase or Moloney murine leukemia virus reverse transcriptase).

Numerous kits can be used to extract RNA *(i.e.,* total RNA including miRNAs) from bodily fluids. Such kits are for example commercially available from, for example, Ambion, Inc. (Austin, TX), Amersham Biosciences (Piscataway, NJ), BD Biosciences Clontech (Palo Alto, CA), BioRad Laboratories (Hercules, CA), GIBCO BRL (Gaithersburg, MD), and Qiagen, Inc. (Valencia, CA). User Guides that describe in great detail the protocol to be followed are usually included in all these kits. Sensitivity, processing time and cost may be different from one kit to another. One of ordinary skill in the art can easily select the kit(s) most appropriate for a particular situation.

In certain embodiments, microRNAs are quantified in HDLs and/or LDLs isolated from the blood sample obtained from the subject to be tested. It is known that microRNAs are transported in plasma and delivered to cells by high-density lipoproteins or low-density lipoproteins (Wang et al., Nucl. Acids Res., 2010, 38: 7248-7259; Arroyo et al., Proc. Natl. Acad. Sci. USA, 2011, 108: 5003-5008; Vickers et al., Nat. Cell Biol., 2011, 13: 423-433). Methods for obtaining circulating lipoproteins (for example by density gradient ultracentrifugation) and analyzing their miRNAs contents are known in the art (Wagner et al., Arterioscler. Thromb. Vase. Biol., 2013, 33: 1392-1400; Niculescu et al., PLoS One, 2015, 10: e0140958).

In other embodiments, microRNAs are quantified in Argonaute protein complexes isolated from the blood sample obtained from the subject to be tested. Methods for obtaining circulating Argonaute protein complexes and analyzing their miRNA contents are known in the art (Turchinovich et al., Methods Mol. Biol., 2013, 1024: 97-107; Hauptmann et al., Proc. Natl. Acad. Sci. USA, 2015, 112(38): 11841-11845; and Flores-Jasso et al., RNA, 2013, 19(2): 271-279)

In yet other embodiments, RNA (including microRNAs) is extracted from exosomes isolated from the blood sample obtained from the subject to be tested. Exosomes are membrane-bound 50-100 nm vesicles released from many cell types. Exosomes transport mainly miRNAs, mRNAs, enzymes, cytokines, etc... from the cells of origin to the neighbor cells mediating the communication between them. In the practice of the present invention, serum exosome isolation may be performed using any suitable method known in the art. Such methods include, but are not limited to, differential ultracentrifugation which generally follows pre-processing protocols (*e.g.,* generation of platelet poor plasma or removal of the 'cell debris'), methods using size-based filters (*e.g.*, ExoMir™, Bioscientific), antibody-based capture (*e.g.*, Immunobeads, HansaBioMed), gel filtration (Sepharose 2B beads, Sigma-Aldrich) and polymer-based precipitation reagents (*e.g.*, Total Exosome Isolation Reagent from Life Technologies, ExoQuik™ from System Biosciences Inc.). Recently developed methods include methods using spin columns (Enderle et al., PLoS One, 2015, 10(8): e0136133), ready-made chromatography columns (Welton et al., J. Extracell. Vesicles, 2015, e:27269. doi: 10.3402/jev.v4.27269; Böing et al., J. Extracell. Vesicles., 2014, 3: 23430), and Protein Organic Solvent Precipitation (PROSPR) (Gallart-Palau et al., Sci. Rep., 2015, 5: 14664). Prior to micro-RNA extractions, the isolated exosomes may be characterized by immunoblotting and transmission electron microscopy.

## B. Detection and Quantification of micro-RNA Biomarkers

In the methods of the invention, the micro-RNA biomarkers may be detected and their levels may be quantified using any suitable method known in the art. There are numerous ways known in the art of determining the level of a micro-RNA in a biological sample (see, for example, Varallyay et al., Methods, 2007, 43: 140-145; Ach et al., BMC Biotechnol., 2008, 8: 69; Kauppinen et al., Methods Mol. Biol., 2008, 451: 217-227; Varallyay et al., Nat. Protoc., 2008, 3: 190-196; Li et al., Anal. Bioanal. Chem., 2009, 394: 1117-1124; Li et al., Anal. Chem., 2009, 81: 5446-5451; Koscianska et al., BMC Mol. Biol., 2011, 12: 14; Roy et al., Lab Chip., 2011, 11: 1886-1894; Baker et al., Nucl. Acids Res., 2012, 40(2):e13; Planell-Saquer et al., Clin. Biochem., 2013, 46: 869-878; He et al., Chem. Commun., 2014, 50: 6236-6239; Tian et al., Org. Biomol. Chem., 2015, 13: 2226-2238; Urbanek et al., Int. J. Mol. Sci., 2015, 16: 13259-13286). Examples of such methods include, but are not limited to, Northern blotting, microRNA arrays, RT-PCR (reverse transcription polymerase chain reaction) methods or other methods of amplification (such as ligase chain reaction (LCR), transcription-mediated amplification (TMA), strand displacement amplification (SDA) and nucleic acid sequence based amplification (NASBA)), next generation sequencing, RNA interference, RNase protection methods, etc...

In Northern Blotting, hybridization may be performed using a complementary $^{32}$P digoxigenin-labeled oligonucleotide or modified Locked-nucleic acid (LNA) oligonucleotides after gel separation. The levels of microRNAs may alternatively be measured using a modified invader assay (Allawi et al., RNA, 2004, 10: 1153-1161), which is based on enzymatic cleavage by a structure-specific 5' nuclease (Cleavase) of a synthetic oligonucleotide (the probe) that is in an appropriate overlap-flap structure. Another widely used technique for detection and profiling of microRNAs is the use of oligonucleotide micro-array based detection platforms either with DNA capture probes or using modified Locked-nucleic-acid (LNA) oligonucleotides in which the ribose moiety is modified with an extra bridge that connects the 2'-O and 4'-C atoms.

In certain preferred embodiments, the level of a microRNA biomarker of the present invention is measured using a quantitative reverse-transcriptase polymerase chain reaction (qRT-PCR). This method is sensitive and requires low amounts of starting material for the detection of individual mature microRNAs. Different variations of qRT-PCR exist for the detection and quantification of miRNAs. In these methods, the miRNAs are usually first extended or used as templates to generate longer sequences that can be amplified by polymerase. These methods include, but are not limited to, RNA tailing (Shi et al., BioTechniques, 2005, 39: 519-525), step-loop primer transcription (Chen et al., Nucleci Acids Res., 2005, 33: e179; Varkonyi-Gasic et al., Plant Methods, 2007, 3: 12; Schmittgen et al., Methods, 2008, 44: 31-38; Siva et al., Mol. Cancer, 2009, 8: 17; Yang et al., Plant Biotechnol. J., 2009, 7: 621-630; Wan et al., RNA, 2010, 16: 1436-1445), adaptor ligation (Lu et al., J. RNAi Gene Silencing, 2005, 1: 44-49), tailed primer extension (Raymond et al., RNA, 2005, 11: 1737-1744; Sharbati-Tehrani et al., BMC Mol. Biol., 2008, 9: 34; Yao et al., RNA, 2009, 15: 1787-1794), padlock probe ligation (Jonstrup et al., 2006, 12: 1747-1752), dual probe ligation (Li et al., Anal. Chem., 2009, 81: 5446-5451; Duncan et al., Anal. Biochem., 2006, 359: 268-270), dumbbell-shaped DNA probe-directed rolling circle amplification (Zhou et al., Nucleic Acids Res., 2010, 38: e156), two microRNA-specific DNA primers (WO 2010/085966; Balcells et al., BMC Biotechnology, 2011, 11: 70; Cirera et al., Methods Mol. Biol., 2014, 1182: 73-81) and pincer probes (Huang et al., PLoS One, 2015, 10: e0120160).

In certain preferred embodiments, the qRT-PCR method used to quantify a micro-RNA biomarker of the present invention is a stem-loop qRT-PCR method. A stem-loop qRT-PCR method includes two steps: miRNA specific stem-loop primer-based reverse transcription (wherein a stem-loop primer is hybridized to the microRNA molecule to be detected, then reverse transcribed with reverse transcriptase enzymes), and then quantification of the reverse transcription products with real-time PCR (*e.g.*, using a conventional Taqman™ assay). A simplified step of stem-loop qRT-PCR uses a stem-loop specific UPL probe (Universal Probe Library) during the quantification of reverse transcription products without decreasing specificity or efficiency (Varkonyi-Gasic et al., Plant Methods, 2007, 3:12).

When using hybridization and/or amplification techniques, the methods of the present invention comprise a step of contacting the biological sample obtained from the subject to be tested with at least one primer or probe that is specific of the micro-RNA biomarker to be quantified.

*Biomarker-Specific Probes and/or Primers*

As used herein, the term *"biomarker-specific probe or primer"* refers to a reagent that is used to detect and quantify a microRNA biomarker of the invention. Examples of such reagents include probes that specifically hybridize to a microRNA biomarker of the invention and PCR primers that specifically amplify at least one region of a microRNA biomarker of the invention. In certain embodiments, the biomarker-specific probe or primer is labeled with a detectable moiety.

Using the miR-7a, miR-215 and miR-375 sequence information provided herein or any other miR-7a, miR-215 and miR-375 sequence information available in the art, one skilled in the art knows how to develop detection probes or amplification primers that are specific for miR-7a, miR-215 or miR-375. Since miR-7a, miR-215 and miR-375 are conserved in many species, the probes or primers may be designed to be usable for more than one mammal species. Methods exist for designing primers for microRNA-specific quantitative qRT-PCR (Busk, BMC Bioinformatics, 2014, doi: 10.1186/1471-2105-15-29), including for designing microRNA-specific stem-loop primers (Czimmerer et al., PLoS One, 2013, 8(1): e55168).

The primers and probes useful in the practice of the present invention are generally oligonucleotides comprising at least 15 nucleotides.

When the oligonucleotide is used as a primer, its length is usually 15 to 100 nucleotides, preferably 17 to 30 nucleotides. In certain embodiments, a pair of PCR primers is used that comprises: a 5' (upstream) primer (or forward primer) that hybridizes with the 5' end of the nucleic acid sequence to be amplified and a 3' (downstream) primer (or reverse primer) that hybridizes with the complement of the sequence to be amplified. Such primer pairs are particularly useful in PCR amplification reactions. In addition to a nucleotide sequence identical or complementary to the nucleotide sequence of the target sequence, the primer may comprise any nucleotide sequence added thereto. Furthermore, the primer may be modified. For example, primers may be labeled with a detectable agent or moiety (*e.g.*, a fluorescent substance or a binding affinity substance such as biotin or digoxin).

These primer pairs can be used according to any nucleic acid amplification technique that employs two or more oligonucleotides to amplify a target sequence. Amplification products produced using a primer pair may be detected using any of a variety of detection methods well known in the art. For example, amplification products may be detected using agarose gel electrophoresis and visualization by ethidium bromide staining and exposure to ultraviolet (UV) light or by sequence analysis of the amplification product, or any other method known in the art.

In embodiments where a qRT-PCR method is employed to determine the level of a microRNA biomarker of the invention, two microRNA-specific primers (one forward primer and one reverse primer) may be used. The sequence of the forward primer may be identical to 12 to 18 nucleotides of the microRNA biomarker and may include a tag at the 5' end; and the reverse primer may be complementary to the 3 to 8 nucleotides of the microRNA followed by 15 T residues and a tail of varying length, wherein the 15 Ts and the tail are identical to part of the reverse transcription primer sequence.

In embodiments where a stem-loop qRT-PCR method is employed to determine the level of a microRNA biomarker of the invention, a miRNA-specific stem-loop reverse transcription primer is used that hybridizes to the target microRNA. This is followed by reverse transcription of the target microRNA resulting in extension of the 3'end of the primer. The result of the reverse transcription is a chimeric (DNA) amplicon with the stem-loop primer sequence at the 5' end of the amplicon and the cDNA of the target microRNA at the 3' end. Quantification of the target microRNA is achieved by real time RT-PCR using a universal reverse primer having a sequence that is complementary to a sequence at the 5' end of the miR-specific stem-loop reverse transcription primer, a target miR-specific forward primer, and a target microRNA sequence-specific TaqMan™ probe.

As used herein, the term *"miRNA-specific stem-loop reverse transcription primer"* refers to a molecule comprising a 3' miRNA-specific portion, a stem, and a loop as described in U.S. Pat. No. 7,575,863 (to Chen et al.). The term "3' *miRNA-specific portion"* refers to a single stranded portion of the miRNA-specific stem-loop reverse transcription primer that is complementary to the micro-RNA biomarker to be detected. The 3' miRNA-specific portion is located downstream from the stem of the miRNA-specific stem-loop reverse transcription primer. Generally, the 3' miRNA-specific portion is between 6 and 9 nucleotides long. In some embodiments, the 3' miRNA-specific portion is 7 nucleotides long. It will be appreciated that routine experimentation can produce other lengths, and that 3' miRNA-specific portions that are longer than 8 nucleotides or shorter than 6 nucleotides are also contemplated by the present teachings. Generally, the 3'-most nucleotides of the 3' miRNA-specific portion should have minimal complementary overlap, or no overlap at all, with the 3' nucleotides of the forward primer; it will be appreciated that overlap in these regions can produce undesired primer dimer amplification products in subsequent amplification reactions. In some embodiments, the overlap between the 3'-most nucleotides of the 3' miRNA-specific portion and the 3' nucleotides of the forward primer is 0, 1, 2 or 3 nucleotides. In some embodiments, greater than 3 nucleotides can be complementary between the 3'-most nucleotides of the 3' miRNA-specific portion and the 3' nucleotides of the forward primer, but generally such situations will be accompanied by additional non-complementary nucleotides interspersed therein. In some embodiments, modified bases such as LNA can be used in the 3' miRNA-specific portion to increase the melting temperature Tm of the stem-loop primer. In some embodiments, universal bases can be used. In some embodiments, modifications including, but not limited to, LNAs and universal bases can improve reverse transcription specificity and potentially enhance detection specificity. The term *"stem"* refers to the double stranded region of the miRNA-specific stem-loop reverse transcription primer that is between the 3' miRNA-specific portion and the loop. As used herein, the term "stem" refers to the double-stranded region of the miRNA-specific stem-loop reverse transcription primer. The stem can be between 6 and 14 base-pairs in length. Generally, one of the complementary strands of the stem can comprise an identifying portion, referred to as "tag", "identifier tag", or "zip-code". Examples of tags may be found in U.S. Pat. No. 6,309,829; U.S. Pat. No. 5,981,176; U.S. Pat. No. 5,935,793; and PCT Publication No. WO 01/92579. The term *"loop"* refers to a region of the miRNA-specific stem-loop reverse transcription primer that is located between the two complementary strands of the stem. Typically, the loop comprises single stranded nucleotides, though other moieties including modified DNA or RNA, carbon spacers such as C18, and/or PEG (polyethylene glycol) are also possible. Generally, the loop is between 4 and 30 nucleotides long. In some embodiments, the loop is between 14 and 18 nucleotides long, for example the loop may be 16 nucleotides long. Generally, the loop will be at least long enough to include sufficient sequence information to encode a promoter. In some embodiments, some of the sequence information encoding the promoter can also reside in the stem.

When the oligonucleotide is used as a probe, its length is usually 5 to 200 nucleotides, preferably 7 to 100 nucleotides, more preferably 7 to 50 nucleotides. The probe is not particularly limited as long as it is capable of specifically hybridizing to a portion of a micro-RNA biomarker of the invention. From the sequence information provided above, one skilled in the art knows how to design and prepare suitable probes.

Generally probes have a complementary nucleic acid sequence that selectively hybridizes to the target nucleic acid sequence. In order for a probe to hybridize to a target sequence, the hybridization probe must have sufficient identity with the target sequence, *i.e.*, at least 70% (*e.g.*, 80%, 90%, 95%, 98% or more) identity with the target sequence. The probe sequence must also be sufficiently long so that the probe exhibits selectivity for the target sequence over non-target sequences.

An oligonucleotide useful in the practice of the present invention (primer or probe) may include one or more sequences which can serve as spacers, linkers, sequences for labeling or binding to an enzyme, which may impart added stability or susceptibility to degradation process or other desirable property to the oligonucleotide.

*Preparatiort of Primers and Probes.* The primers and probes that can be used in the practice of the present invention may be prepared using any of a variety of methods well known in the art (see, for example, J. Sambrook et al., "Molecular Cloning: A Laboratory Manual", 2012, 4th Ed., Cold Spring Harbour Laboratory Press: New York, NY; *"*PCR Protocols: A Guide to Methods and Applications", 1990, M.A. Innis (Ed.), Academic Press: New York, NY; P. Tijssen "Hybridization with Nucleic Acid Probes - Laboratory Techniques in Biochemistry and Molecular Biology (Parts I and II)", 1993, Elsevier Science; "PCR Strategies", 1995, M.A. Innis (Ed.), Academic Press: New York, NY; and *"*Short Protocols in Molecular Biology", 2002, F.M. Ausubel (Ed.), 5th Ed., John Wiley & Sons: Secaucus, NJ). For example, oligonucleotides may be prepared by chemical synthesis and polymerization based on a template as described, *e.g.,* in S.A. Narang et al., Meth. Enzymol. 1979, 68: 90-98; E.L. Brown et al., Meth. Enzymol. 1979, 68: 109-151; E.S. Belousov et al., Nucleic Acids Res. 1997, 25: 3440-3444; D. Guschin et al., Anal. Biochem. 1997, 250: 203-211; M.J. Blommers et al., Biochemistry, 1994, 33: 7886-7896; and K. Frenkel et al., Free Radic. Biol. Med. 1995, 19: 373-380; and U.S. Pat. No. 4,458,066).

For example, oligonucleotides may be prepared using an automated, solid-phase procedure based on the phosphoramidite approach. Such syntheses may be performed on oligo synthesizers such as those commercially available from Perkin Elmer/Applied Biosystems, Inc. (Foster City, CA), DuPont (Wilmington, DE) or Milligen (Bedford, MA). Alternatively, oligonucleotides can be custom made and ordered from a variety of commercial sources well-known in the art, including, for example, the Midland Certified Reagent Company (Midland, TX), ExpressGen, Inc. (Chicago, IL), Operon Technologies, Inc. (Huntsville, AL), BioSearch Technologies, Inc. (Novato, CA), and many others.

Purification of oligonucleotides of the invention, where necessary or desired, may be carried out by any of a variety of methods well known in the art. Purification of oligonucleotides is typically performed either by native acrylamide gel electrophoresis, by anion-exchange HPLC as described, for example, by Pearson et al. (J. Chrom., 1983, 255: 137-149) or by reverse phase HPLC (McFarland et al., Nucleic Acids Res., 1979, 7: 1067-1080).

The sequence of oligonucleotides can be verified using any suitable sequencing method including, but not limited to, chemical degradation (Maxam et al., Methods of Enzymology, 1980, 65: 499-560), matrix-assisted laser desorption ionization time-of-flight (MALDI-TOF) mass spectrometry (Pieles et al., Nucleic Acids Res., 1993, 21: 3191-3196), mass spectrometry following a combination of alkaline phosphatase and exonuclease digestions (Wu et al., Anal. Biochem., 2001, 290: 347-352), and the like.

*Labelling of Primers and/or Probes.* In certain embodiments, detection probes or amplification primers or both probes and primers are labeled with a detectable agent or moiety before being used in amplification/detection assays. In some preferred embodiments, the detection probes are labeled with a detectable agent. The role of a detectable agent is to allow visualization and detection of amplified target sequences (amplicons). Preferably, the detectable agent is selected such that it generates a signal which can be measured and whose intensity is related (*e.g.*, proportional) to the amount of amplification products in the sample being analyzed.

The association between an oligonucleotide (*e.g.*, detection probe) and a detectable agent can be covalent or non-covalent. Labeled detection probes can be prepared by incorporation of, or conjugation to, a detectable moiety. Labels can be attached directly to the nucleic acid sequence or indirectly (*e.g.*, through a linker). Linkers and spacer arms of various lengths are known in the art and are commercially available, and can be selected to reduce steric hindrance, or to confer other useful or desired properties to the resulting labeled molecules (see, for example, Mansfield et al., Mol. Cell Probes, 1995, 9: 145-156).

Methods for labeling nucleic acid molecules are well-known in the art. For a review of labeling protocols and label detection techniques, see, for example, Kricka, Ann. Clin. Biochem. 2002, 39: 114-129; van Gijlswijk et al., Expert Rev. Mol. Diagn. 2001, 1: 81-91; and Joos et al., J. Biotechnol. 1994, 35: 135-153. Standard nucleic acid labeling methods include: incorporation of radioactive agents, direct attachments of fluorescent dyes or of enzymes; chemical modifications of nucleic acid molecules making them detectable immunochemically or by other affinity reactions; and enzyme-mediated labeling methods, such as random priming, nick translation, PCR and tailing with terminal transferase (for a review on enzymatic labeling, see, for example, Temsamani et al., Mol. Biotechnol. 1996, 5: 223-232). Other nucleic acid labeling systems include, but are not limited to: ULS (Universal Linkage System), which is based on the reaction of monoreactive cisplatin derivatives with the N7 position of guanine moieties in DNA (Heetebrij et al., Cytogenet. Cell. Genet. 1999, 87: 47-52), psoralen-biotin, which intercalates into nucleic acids and upon UV irradiation becomes covalently bonded to the nucleotide bases (Levenson et al., Methods Enzymol. 1990, 184: 577-583; and Pfannschmidt et al., Nucleic Acids Res. 1996, 24: 1702-1709), photoreactive azido derivatives (Neves et al., Bioconjugate Chem. 2000, 11: 51-55), and DNA alkylating agents (Sebestyen et al., Nat. Biotechnol. 1998, 16: 568-576).

Any of a wide variety of detectable agents can be used in the practice of the present invention. Suitable detectable agents include, but are not limited to, various ligands, radionuclides (such as, for example, $^{32}$P, $^{35}$5, $^{3}$H, $^{14}$C, $^{125}$I, $^{131}$I, and the like); fluorescent dyes (see below); chemiluminescent agents (such as, for example, acridinium esters, stabilized dioxetanes, and the like); spectrally resolvable inorganic fluorescent semiconductor nanocrystals (*i.e.*, quantum dots), metal nanoparticles (*e.g.*, gold, silver, copper and platinum) or nanoclusters; enzymes (such as, for example, those used in an ELISA, *i.e.,* horseradish peroxidase, beta-galactosidase, luciferase, alkaline phosphatase); colorimetric labels (such as, for example, dyes, colloidal gold, and the like); magnetic labels (such as, for example, Dynabeads™); and biotin, dioxigenin or other haptens and proteins for antisera or monoclonal antibodies are available.

In certain embodiments, the detection probes are fluorescently labeled. Numerous known fluorescent labeling moieties of a wide variety of chemical structures and physical characteristics are suitable for use in the practice of this invention. Suitable fluorescent dyes include, but are not limited to, fluorescein and fluorescein dyes (*e.g.*, fluorescein isothiocyanine or FITC, naphthofluorescein, 4',5'-dichloro-2',7'-dimethoxy-fluorescein, 6-carboxyfluorescein or FAM), carbocyanine, merocyanine, styryl dyes, oxonol dyes, phycoerythrin, erythrosin, eosin, rhodamine dyes (*e.g.*, carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (*e.g.*, methoxycoumarin, dialkylaminocoumarin, hydroxycoumarin and amino-methylcoumarin or AMCA), Oregon Green Dyes (*e.g.,* Oregon Green 488, Oregon Green 500, Oregon Green 514), Texas Red, Texas Red-X, Spectrum Red™, Spectrum Green™, cyanine dyes (*e.g.*, Cy-3™, Cy-5™, Cy-3.5™, Cy-5.5™), Alexa Fluor dyes (*e.g.*, Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (*e.g.*, BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (*e.g.*, IRD40, IRD 700, IRD 800), and the like. For more examples of suitable fluorescent dyes and methods for linking or incorporating fluorescent dyes to nucleic acid molecules see, for example, "The Hanlbook of Fluorescent Probes and Research Products", 9th Ed., Molecular Probes, Inc., Eugene, OR. Fluorescent dyes as well as labeling kits are commercially available from, for example, Amersham Biosciences, Inc. (Piscataway, NJ), Molecular Probes Inc. (Eugene, OR), and New England Biolabs Inc. (Berverly, MA).

Rather than being directly detectable themselves, some fluorescent groups (donors) transfer energy to another fluorescent group (acceptor) in a process of fluorescent resonance energy transfer (FRET), and the second group produces the detectable fluorescent signal. In these embodiments, the oligonucleotide detection probe may, for example, become detectable when hybridized to an amplified target sequence. Examples of FRET acceptor/donor pairs suitable for use in the present invention include, but are not limited to, fluorescein/tetramethylrhodamine, IAEDANS/FITC, IAEDANS/5-(iodoacetomido)fluorescein, B-phycoerythrin/Cy-5, FAM/TAMRA and EDANS/Dabcyl.

The use of physically linked fluorescent reporter/quencher molecule pairs is also within the scope of the invention. The use of such systems in TaqMan™ assays or as Molecular Beacons is well-known in the art. With the TaqMan™ assay format, products of the amplification reaction can be detected as they are formed in a so-called "real-time" manner. As a result, amplification product/probe hybrids are formed and detected while the reaction mixture is under amplification conditions.

In some embodiments of the present invention, the PCR detection probes are TaqMan™-like probes that are labeled at the 5'-end with a fluorescent moiety and at the 3'-end with a quencher moiety. Suitable fluorophores and quenchers for use with TaqMan™-like probes are known in the art. Examples of quenchers include, but are not limited to DABCYL (*i.e.*, 4-(4'-dimethylaminophenylazo)-benzoic acid) succinimidyl ester, diarylrhodamine carboxylic acid, succinimidyl ester (or QSY-7), and 4',5'-dinitrofluorescein carboxylic acid, succinimidyl ester (or QSY-33) (all available, for example, from Molecular Probes), quencher1 (Q1; available from Epoch Biosciences, Bothell, WA), or "Black hole quenchers" BHQ-1, BHQ-2, and BHQ-3 (available from BioSearch Technologies, Inc., Novato, CA).

A "tail" of normal or modified nucleotides can also be added to oligonucleotide probes for detectability purposes. A second hybridization with a nucleic acid molecule complementary to the tail and containing one or more detectable labels allows visualization of the amplicon/probe hybrids (see, for example, the system commercially available from Enzo Biochem, Inc. New York, NY). Another example of an assay with which the inventive oligonucleotides are useful is a signal amplification method such as that described in U.S. Pat. No. 5,124,246. In that method, the signal is amplified through the use of amplification multimers, polynucleotides which are constructed so as to contain a first segment that hybridizes specifically to the "tail" added to the oligonucleotide probes, and a multiplicity of identical second segments that hybridize specifically to a labeled probe. The degree of amplification is theoretically proportional to the number of iterations of the second segment. The multimers may be either linear or branched. Branched multimers may be in the shape of a fork or a comb.

The selection of a particular nucleic acid labeling technique will depend on the situation and will be governed by several factors, such as the ease and cost of the labeling method, the quality of sample labeling desired, the effects of the detectable moiety on the hybridization reaction *(e.g.,* on the rate and/or efficiency of the hybridization process), the nature of the amplification method used, the nature of the detection system, the nature and intensity of the signal generated by the detectable label, and the like.

*Immobilization of Probes.* In certain embodiments, a detection probe is immobilized on a solid support. For example, the solid support may be a bead, a particle, a microplate well, an array, a cuvette, a tube, a membrane, a gel, a resin, and the like.

In certain embodiments, an inventive probe is immobilized by being either covalently or non-covalently bound to the surface of a solid carrier or support. The term *"solid support"* refers to a material to which nucleic acid molecules can be immobilized. The solid support is not particularly limited as long as it is capable of immobilizing nucleic acid molecules. Thus, in the practice of the present invention, the solid support may be any solid support known in the art to which the probe can be operably affixed. *"Operably affixed"* refers to the probe being affixed in a manner permitting the formation of a complex between the affixed probe and the amplicons (or the target sequence) present in the biological sample tested. Examples of suitable carrier or support materials include, but are not limited to, agarose, cellulose, nitrocellulose, dextran, Sephadex, Sepharose, carboxymethyl cellulose, polyacrylamides, polystyrene, polyvinyl chloride, polypropylene, gabbros, magnetic ion-exchange resin, glass, polyamine-methyl-vinyl-ether-maleic acid copolymer, amino acid copolymer, ethylene-maleic acid copolymer, nylon, silk, and the like. In certain embodiments, immobilization of a probe to a solid carrier or support includes gel electrophoresis followed by transfer to a membrane (typically nitrocellulose or PVDF) in a process called western blotting (or immunoblot) well known in the art.

Methods for generating arrays are known in the art and include, *e.g.*, photolithographic methods, mechanical methods, pin-based methods, and bead-based techniques. Oligonucleotide probes forming an array may be attached to a substrate by any number of techniques, including, without limitation, (i) *in situ* synthesis (*e.g.*, high-density oligonucleotide arrays) using photolithographic techniques; (ii) spotting/printing at medium to low density on glass, nylon or nitrocellulose; (iii) by masking, and (iv) by dot-blotting on a nylon or nitrocellulose hybridization membrane. Oligonucleotides can be immobilized *via* a linker, including by covalent, ionic, or physical linkage. Linkers for immobilizing nucleic acids and polypeptides, including reversible or cleavable linkers, are known in the art.

Instead of being designed and prepared, probes and primers specific for miR-7a, miR-215 and miR-375, including microRNA-specific stem-loop primers may be purchased, for example from Life technologies, Eurogentec, Applied Biosystem, Amplion, Qiagen, Quanta Biosciences, Sigma-Aldrich and Exiqon.

### Contacting Biological Samples with Biomarker-Specific Probes and/or Primers

As used herein, the term *"contacting the biological sample with a biomarker-specific probe or primer"* typically includes, but is not limited to, mixing or incubating the biological sample with a probe or primer that is specific for a micro-RNA biomarker of the invention. Contacting may be performed in any suitable device or container, such as a plate, microtiter dish, test tube, column, well, and the like. In certain embodiments, the contacting is performed on a substrate coated with a reagent, such as a miRNA array. In preferred embodiments, the step of contacting the biological sample is performed for a time and under conditions allowing the probe or primer to react with the micro-RNA biomarker present in the biological sample.

It is within the abilities of one skilled in the art to determine the optimal conditions allowing the primers or probes to react with a micro-RNA biomarker present in the biological sample such that the micro-RNA level can be quantified. In certain embodiments, the conditions are selected such that the biomarker-specific probes can specifically hybridize to the micro-RNA biomarker present in the biological sample, for example under stringent hybridization conditions. In other embodiments, the conditions are selected such that the biomarker-specific PCR primers can specifically amplify at least one portion of the micro-RNA biomarker present in the biological sample.

### *Levels of* micro-RNA *Biomarkers*

In a method of the invention, once the level of a microRNA biomarker has been determined in the biological sample obtained from a subject to be tested, it is compared to the level of the same microRNA biomarker determined in one or more control or reference samples. As will be acknowledged by one skilled in the art, when comparisons of levels of microRNAs measured in different biological samples are performed, the biological samples must have been identically processed. In addition, comparison of levels is preferably preformed after the levels measured have been corrected for both differences in the amount of sample assayed and variability in the quality of the sample used (*e.g.*, volume of blood sample, amount of exosomes isolated, and/or amount and quality of RNA tested). Correction may be carried out using any suitable method well-known in the art. For example, correction may be formed by normalizing the levels measured for the microRNA biomarker against a housekeeping RNA that is constitutively expressed. Suitable RNAs for normalization include housekeeping RNAs such as the U6, U24, and U48 or stable miRNAs that vary very little such as miR18a and miR101 for example and/or spike control. This normalization allows the comparison of miRNA levels in different samples from the same subject, or in samples from different sources (*e.g.*, the subject to be tested and a control subject). Thus, the normalized level of a microRNA biomarker of the invention determined in a biological sample to be tested according to a method described herein may then be compared to the normalized level of the same microRNA biomarker determined in one or more control biological samples (for diagnosis or prognosis purposes - see below) or to the normalized level of the same microRNA biomarker determined in a biological sample obtained from the same subject after receiving a treatment (*e.g.*, for assessment of therapy effectiveness - see below).

In certain embodiments, the control biological sample originates from a healthy subject. In other embodiments, the control biological sample originates from a type 1 prediabetic subject that is known to not have developed type 1 diabetes. In still other embodiments, the control biological sample originates from a subject with a known degree of insulitis. In yet other embodiments, the control biological sample originates from a type 1 diabetic subject. The subject to be tested using a method of the invention and the control subject must be of the same species. In certain preferred embodiments, the subject to be tested and the control subject are of the same sex, and/or of similar age, and/or have similar body mass index and/or ethnic background.

In certain preferred embodiments, normalized levels of a microRNA biomarker of the invention determined in a biological sample to be tested are compared to the average of normalized levels of the same microRNA biomarker determined for biological samples obtained from a population (*i.e.,* from a significant number) of control subjects.

Based on the teaching provided herein, one skilled in the art will know how to select the controls and comparisons to be performed in order to reach a diagnosis or prognosis according to the present invention.

In certain embodiments, the levels of the same microRNA biomarker determined in control or reference samples (from healthy subjects, from prediabetic subjects known to not have developed type 1 diabetes, from subjects with known degree of insulitis, and from type 1 diabetes subjects) are baseline or reference values that have been generated using a method of the invention and that are contained in a machine-readable medium such as for example optical media (CD-ROM, DVD-ROM and the like), and solid state memory. In such embodiments, "comparing the level of a microRNA biomarker determined in a biological sample of a subject to be tested with the level of the same microRNA biomarker determined in a biological sample obtained from at least one control subject" means comparing the level of a microRNA biomarker determined in a biological sample of a subject to be tested with predetermined baseline or reference values.

### C. Uses of Determination of microRNA Biomarkers Levels

The methods of the invention may be used to provide a diagnosis or prognosis regarding the subject tested, and to determine the appropriate course of action for a given patient (*e.g.*, immune intervention strategy). They may also be used to assess the effectiveness of a therapeutic treatment, to monitor the responsiveness of a patient to a therapeutic treatment, or to screen for potentially useful therapeutic compounds.

### *Diagnosis and Prognosis*

In certain embodiments, the level of miR-7a measured in a biological sample obtained from the subject to be tested is compared with the level of miR-7a measured in a biological sample of at least one healthy subject. The subject to be tested is identified as likely of future development of type 1 diabetes if the level of miR-7a measured in the biological sample obtained from the subject to be tested is found to be statistically higher than the level of miR-7a measured for the healthy subject. The subject to be tested is identified as not likely of future development of type 1 diabetes if the level of miR-7a measured in the biological sample obtained from the subject to be tested is not found to be statistically higher *(i.e.,* is found to be similar to or statistically lower) than the level of miR-7a measured for the healthy subject.

In other embodiments, the level of miR-7a measured in a biological sample obtained from the subject to be tested is compared with the level of miR-7a measured in a biological sample of at least one healthy subject. The subject to be tested is diagnosed with insulitis if the level of miR-7a measured in the biological sample obtained from the subject to be tested is found to be statistically higher than the level of miR-7a measured for the healthy subject.

As used herein, the terms *"increase"* or *"higher"* refer to an increase (or augmentation) of at least 5%, at least about 10%, at least about 20%, at least 25%, at least 30%, at least 40%, at least about 50%, at least about 75%, at least about 80%, at least about 100%, at least about 200% (*i.e.,* 2-fold), or at least about 500% (*i.e., 5*-fold), or at least about 1000% *(i.e.,* 10-fold) or more over the level of expression under control conditions.

As used herein, by the terms "*decrease*" or "*lower*" refer to a decrease (or reduction) of at least 5%, at least about 10%, at least about 25%, at least about 50%, at least about 75%, at least about 80%, at least about 90% or less of the level of expression under control conditions.

In yet other embodiments, the levels of miR-7a, miR-215 and miR-375 measured in a biological sample obtained from the subject to be tested and combined together according to the present teaching are compared with the levels of miR-7a, miR-215 and miR-375 measured in a biological sample of at least one healthy subject and combined together according to the present teaching. As used herein, the term *"combined together according to the present teaching"* refers to any combination wherein high levels of miR-7a and miR-215 associated with a low level of miR-375 are correlated with a high degree of insulitis. A combination according to the present teaching may be, for example, the following combination:

$$0.25 \text{ RQ(miR-7a)} + 0.66 \text{ RQ(miR-215)} - 0.42 \text{ RQ(miR-375)},$$

wherein RQ(miR) is the relative (or normalized) level of the miR (see Examples).
Another suitable combination is:

$$0.2 \text{ RQ(miR-7a)} + 0.6 \text{ RQ(miR-215)} - 0.4 \text{ RQ(miR-375)},$$

wherein RQ(miR) is the relative (or normalized) level of the miR (see Examples). An increased combination (or combined value) in the tested subject compared to the healthy subject is indicative of insulitis in the tested subject.

In another aspect, the present invention provides an *in vitro* method for staging or scoring insulitis in a subject. The terms "*insulitis score*" and "*insulitis stage*" are used herein interchangeably. They refer to a value, which defines a discrete point in the course of insulitis, and provides information about the degree of advancement and severity of the disease. Insulitis scores are currently obtained by histological evaluation of islets and are determined as follows: 0 = no intra islet mononuclear cell infiltration, 1 = peri-insulitis, 2 = mild mononuclear infiltration, 3 = moderate mononuclear infiltration and 4 = severe mononuclear infiltration. The stages of mild, moderate and severe infiltration have been interpreted differently in the scientific community. Thus, for example, in Serreze et al. (J. Immunol., 1998, 161: 3913-3918), 2 = <25% of infiltrated islet; 3 = 25-75% of infiltrated islet and 4 = >75% of infiltrated islet; in Kaino et al. (J. Pediatr. Endocrinol. Metab., 1998, 11: 267-272), ), 2 = <1/3 of infiltrated islet; 3 = 1/3 to 1/2 of infiltrated islet and 4 = >50% of infiltrated islet; in Miwako and Shudo (Biol. Pharm. Bull, 2009, 32: 157-159), 2 = <50% of infiltrated islet, 3 = >50% of infiltrated islet and 4 = complete islet destruction; in Goudy et al. (J. Immunol., 2003, 171: 2270-2278), 2 = <25% of infiltrated islet; 3 = 25-50% of infiltrated islet and 4 = >50% of infiltrated islet; while in the present study, 2 = <10% of infiltrated islet, 3 = 10-50% of infiltrated islet, and 4 = > 50% of infiltrated islet (see Examples below).

The present invention provides an *in vitro* method for staging or scoring insulitis in a subject, wherein the method comprises steps of: (a) determining the levels of miR-7a, miR-215 and miR-375 in a biological sample obtained from said subject; and (b) combining the levels of miR-7a, miR-215 and miR-375 to obtain the stage/score of insulitis in said subject. In this method, the score of insulitis (IS) may be calculated using any combination wherein high levels of miR-7a and miR-215 associated with a low level of miR-375 are correlated with a high degree of insulitis.

In certain embodiments, the stage or score of insulitis (IS) may be calculated as:

$$\text{IS} = 0.65 + 0.25 \text{ RQ(miR-7a)} + 0.66 \text{ RQ(miR-215)} - 0.42 \text{ RQ(miR-375)},$$

wherein RQ(miR-7a) is the level of the miR-7a measured in the biological sample, RQ(miR-215) is the level of the miR-215 measured in the biological sample, and RQ(miR-375) is the level of the miR-375 measured in the biological sample. Alternatively, IS may be calculated as:

$$IS = 0.6 + 0.2\,RQ(miR\text{-}7a) + 0.6\,RQ(miR\text{-}215) - 0.4\,RQ(miR\text{-}375),$$

wherein RQ(miR-7a) is the level of the miR-7a measured in the biological sample, RQ(miR-215) is the level of the miR-215 measured in the biological sample, and RQ(miR-375) is the level of the miR-375 measured in the biological sample.

Using a diagnostic/prognostic/staging method described herein, skilled physicians may, based on the diagnosis/prognosis reached, select and prescribe treatments adapted to each patient (for example asymptotic patients who are likely to develop type 1 diabetes *versus* asymptotic patients who will not progress to type 1 diabetes). Selection of an appropriate therapeutic regimen for a given patient may be made solely on the diagnosis/prognosis provided by the inventive methods. Alternatively, the physician may also consider other clinical or pathological parameters.

### Effectiveness of Therapeutic Treatments

A method according to the invention may also be used to assess the efficacy of a therapeutic treatment administered to prevent progression of insulitis or autoimmunity (for example by monitoring the levels of miR-7a, miR-215 and miR-375) or may be used to assess the efficacy of a therapeutic treatment administered to prevent the development of type 1 diabetes or the progression of insulin pancreatic cell aggression at or following type 1 diabetes diagnosis (for example by monitoring the level of miR-7a). Based on the results obtained in a monitoring assay of the invention, a physician may adjust or optimize the dose regimen administered to the patient in order to achieve therapeutic efficacy and/or to reduce side effects. Alternatively, based on the results obtained in a monitoring assay of the invention, a physician may decide to stop administration of the therapeutic treatment.

Generally, a monitoring assay according to the invention comprises steps of: determining the level of miR-7a (or the levels of miR-7a, miR-215 and miR-375) in a biological sample obtained from a patient *(e.g.,* a patient diagnosed as likely to develop type 1 diabetes in the future, or a patient diagnosed with insulitis) after administration of a therapeutic treatment (or during treatment); and comparing the level of miR-7a (or the level of miR-7a, miR-215 and miR-375 combined together according to the present teaching) measured with the level of miR-7a (or the level of miR-7a, miR-215 and miR-375 combined together according to the present teaching) measured in a biological sample obtained from the patient prior to administration of the therapeutic treatment. A statistically significant decrease of the level(s) of miR-7a (or the level of miR-7a, miR-215 and miR-375 combined together according to the present teaching) during or after treatment is indicative of an effective therapeutic treatment. In contrast, constant or increasing levels of miR-7a (or the level of miR-7a, miR-215 and miR-375 combined together according to the present teaching) during or after treatment are indicative of an ineffective therapeutic treatment.

### Screening Methods

A method according to the invention may also be used to identify compounds with the ability to prevent progression of insulitis (for example by assessing the effect of a candidate compound on the levels of miR-7a, miR-215 and miR-375 combined together according to the present teaching) or to identify compounds with the ability to prevent the development of type 1 diabetes (for example by assessing the effect of a candidate compound on the level of miR-7a).

A screening method of the invention may be performed on an animal model *(e.g.,* the non-obese diabetic (NOD) mouse - an animal model of spontaneous type 1 diabetes), or in a cell or cell line, such as insulin cells, insulinoma cell lines (for example Min6, INSI, RIN...) or immune cells or cell lines (for example, lymphocytes, dendritic cells, macrophages/monocytes, NK cells, RAW264.7, EL-4, JAWSII..).

Generally, a screening assay according to the invention comprises steps of: determining the level of miR-7a (or the levels of miR-7a, miR-215 and miR-375) in a cell line or in an animal model after contacting the cell line or after administering the animal model with a candidate compound; and comparing the level of miR-7a (or the level of miR-7a, miR-215 and miR-375 combined together according to the present teaching) measured with the level of miR-7a 7a (or the level of miR-7a, miR-215 and miR-375 combined together according to the present teaching) in the cell line or animal model prior to contacting or administration with the candidate compound. A statistically significant decrease of the level(s) of miR-7a or of the described combination of miR-7a, miR-215 and miR-375 during or after treatment is indicative of an effective candidate compound. In contrast, constant or increasing levels or miR-7a or the described combination of miR-7a, miR-215 and miR-375 during or after treatment are indicative of an ineffective candidate compound.

### III - Diagnostic Kits

In another aspect, the present invention provides kits comprising materials useful for carrying out a diagnostic/prognostic method of the invention. The diagnostic/prognostic procedures described herein may be performed by analytical laboratories, research laboratories and practitioners. The invention provides kits that can be used in these different settings.

Materials and reagents for performing a diagnostic/prognostic method of the present invention may be assembled together in a kit. In certain embodiments, an inventive kit comprises at least one probe and/or primer specific for miR-7a, allowing the level of miR-7a to be measured in a biological sample. In other embodiments, an inventive kit comprises at least one probe and/or primer for each of the three micro-RNAs: miR-7a, miR-215 and miR-375, allowing the level of each of the three micro-RNAs to be measured in a biological sample. As indicated above, the primers and/or probes may be attached to a solid support (*e.g.,* a macro- or micro-array) and/or labeled with a detectable moiety.

When the kit is to be used in a miRNA-specific stem-loop qRT-PCT method, the kit may comprise a miRNA-specific stem-loop primer, a universal reverse primer having a sequence that is complementary to a sequence at the 5' end of the stem-loop miRNA-specific primer, a miRNA-specific forward primer, and a miRNA-specific TaqMan™ probe.

Depending on the procedure, the kit may further comprise one or more of: extraction buffer and/or reagents, washing buffer and/or reagents, amplification buffer and/or reagents, hybridization buffer and/or reagent, labelling buffer and/or reagents, and detection means. Protocols for using these buffers and reagents to perform different steps of the diagnostic/prognostic procedure may be included in the kit. Other reagents can be included in separate containers and provided with the kit, *e.g.,* positive samples or compounds, negative samples or compounds, buffers, cell culture media, specific detection probes, and the like. A kit according to the present invention may also comprise reagents for normalizing the miRNA levels measured, including spikes for technical internal control and/or reagents for measuring housekeeping RNAs (such as U6, U24 or U48) or reagents for measuring stable miRNAs that vary very little (such as miR18a or miR101).

The reagents may be supplied in a solid (*e.g.*, lyophilized) or liquid form. The kits of the present invention may optionally comprise different containers (*e.g.*, vial, ampoule, test tube, flask or bottle) for each individual buffer and/or reagent. Each component will generally be suitable as aliquoted in its respective container or provided in a concentrated form. Other containers (*e.g.*, a micro-titer plate) suitable for conducting certain steps of the disclosed methods may also be provided. The individual containers of the kit are preferably maintained in close confinement for commercial sale.

A kit according to the present invention may further comprise instructions for using the kit according to a method of the invention. Instructions for using the kit according to a method of the invention may comprise instructions for processing the biological sample obtained from the subject to be tested, and/or instructions for contacting the sample with a microRNA-specific probe or primer, and/or instructions for labelling the primers and/or probes (if they are not pre-labelled), and/or instructions for performing the test, and/or instructions for interpreting the results. A kit may also contain a software package enabling statistical analysis through necessary correction and normalization.

Optionally associated with the container(s) can be a notice or package insert in the form prescribed by a governmental agency regulating the manufacture, use or sale of pharmaceutical or biological products, which notice reflects approval by the agency of manufacture, use or sale for human administration.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

The invention will be further illustrated by the following figures and examples. However, these examples and figures should not be interpreted in any way as limiting the scope of the present invention.

### Brief Description of the Drawing

**Figure 1.** Correlation of the degree of insulitis with serum miR-7a relative expression levels in prediabetic NOD mice (early moderate insulitis; n=10, Rho Spearman = 0.8909, p=0.0011, Prism software, GraphPad).

**Figure 2.** MiR-7a is detected in higher relative quantity in the serum of NOD mice during the 3 months preceding the onset of type 1 diabetes (n=5 mice that become diabetic vs. 2 control mice that remain non-diabetic during the analyzed period of 10 months, p=0.0476, Mann-Whitney, Medcalc end Prism softwares).

**Figure 3.** ROC curve for the detection of serum miR-7a relative expression in prediabetic NOD mice (n=7 - see Figure 2, area under the curve 0.875, p=0.083, Prism software, GraphPad).

**Examples**

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually performed or data were actually obtained.

Materials and Methods

*Mice and Diabetes*

NOD/ShiLtJ female mice were purchased from Charles River Laboratories (L'Arbresles, France). Mice were considered diabetic when blood glucose levels were higher than 11 mM in two consecutive assays. Diabetes incidence in the NOD mouse colony used was 75 % that of diabetic female mice at 40 weeks of age. All cares and experiments with mice were carried out in strict accordance with relevant French guidelines (Décret 2001- 464 of may 29, 2001 and Décret 2013-118 of February 1, 2013). Animals were housed in the Oniris Vet. School Rodent Facility (Agreement Number: 44 266) in a specific pathogen-free environment with sterilized tap water and food. Procedures on animals were approved by the Pays de la Loire regional Committee on the Ethics of Animal Experiments (Permit Number: CEEA.2012.251). All efforts were made to minimize suffering. To obtain the serum, blood was collected onto dry tubes. After 30 minutes of coagulation at room temperature, the serum was separated by centrifugation at 5000xg, 16°C during 5 minutes. Only sera without hemolysis were included in the study, because it has been shown that the microRNAs of erythrocytes influence the profile of the circulating microRNAs (Chen *et al.*, PLoS One, 2008, 3(6): e2360).

*Insulitis Scoring*

Sections of 7 $\mu$m of frozen pancreata were stained with hematoxylin and eosin and the degree of insulitis was rated independently by two investigators in a blinded manner on a total of at least 100 islets. Histological scoring of insulitis in pancreata was performed as follows: 0=islet free of mononuclear cell infiltration, 1=peri-insulitis, 2 to 4=infiltration by immune cells involving less than 10%, from 10% to 50%, and more than 50% of the islet area, respectively. For each pancreas, an average insulitis score was calculated by adding the products of the number of islets x score of each islet and dividing by the total number of islets counted.

*Extraction of RNA and Quantification by Real Time Analysis PCR*

The extraction of serum miRNAs and their quantification were realized for all the samples simultaneously in order to limit the introduction of inter-assay variations. RNA was extracted from 50 $\mu$l of serum using TriReagent (SIGMA, saint Quentin-Fallavier, France) according to the instructions of suppliers modified as follows: (1) addition in the serum of 1010 copies of a synthetic analogue of the microRNA athmiR-159a (Eurogentec, Angers, France) as internal control; (2) use of 500 ng of tRNA of yeast to facilitate the precipitation of RNAs (Sigma); and (3) mixing RNAs with 30 $\mu$l of 10 mM TRIS-Cl, 1 mM EDTA, 0,1% BSA buffer. RNAs were then processed for reverse transcription without further purification. Quantification of miRNAs was carried out using RT-stem-loop primers and TaqMan assays (Life Technologies, Saint Aubin, France). A 12 cycle pre-amplification step was included prior to real-time PCR quantification on an ABI7300 instrument (Life Technologies) using Solis BioDyne reagents (Tartu, Estonie). Relative quantities of tested miR were normalized to miR18a and miR101 and median centered.

*Statistical Analysis*

Statistical analyses were performed using MedCalc Software or Prism (GraphPad Software, Inc.) and statistical tests as indicated in then figure legends.

Results

Six microRNAs were studied in the serum of female prediabetic NOD mice (non-obese diabetic, developing spontaneously type 1 diabetes) presenting various stages or scores of insulitis (autoimmune infiltration of the endocrine pancreas). These microRNAs were chosen wince they have previously been found to be of biological interest by the Inventors (Salama *et al.*, PloS One, 2004, 9: e106153) or following a preliminary study of the serum miRNome in eight NOD mice by TLDA chips (TaqMan Low Density Array analysis testing more than 640 murine microRNAs, data not shown). The study showed the interest of serum microRNAs as predictive biomarkers of pre-diabetes and type 1 diabetes.

First, the present Inventors showed that the age of the mice was not correlated to serum relative expression levels of the six tested microRNAs, thus allowing the use of these microRNAs for their predictive value, independently of the age of individuals (Table 1).

**Table 1:** Absence of correlation between the age of the tested mice and the relative quantities of tested serum miRNAs (n=25 excepted for miR-7a where n=24) (Spearman, Prism Software, GraphPad).

| | A | B | C | D | E | F |
|---|---|---|---|---|---|---|
| | Age (weeks) vs. miR | | | | | |
| | miR-7a | miR-375 | miR-467a | miR-215 | Let-7e | miR-29b |
| | Y | Y | Y | Y | Y | Y |
| 95% confidence interval | -0.3769 to 0.4497 | -0.2705 to 05249 | -0.6904 to 0.01166 | -0.3442 to 0.4634 | -0.09357 to 0.6449 | -0.6723 to 0.04551 |
| | | | | | | |
| P value | | | | | | |
| P (two tailed) | 0.8386 | 0.4693 | 0.0502 | 0.7349 | 0.1138 | 0.0713 |
| P value summary | ns | ns | ns | ns | ns | ns |
| Exact (E) or approximate (A) P value? | A | A | A | A | A | A |
| Significant? (alpha = 0.05) | No | No | No | No | No | No |
| | | | | | | |
| Number of XY pairs | 24 | 25 | 25 | 25 | 25 | 25 |

Secondly, combined expression of three of the six microRNAs tested (namely miR-7a, miR-215 and miR-375) was found to be associated to the degree of insulitis in prediabetic NOD mice (n=24, multivariable regression, analyze of variance p=0.0460, Table 2). The regression equation was found to be:

$$IS = 0.6535 + 0.2542 \, RQ(miR\text{-}7a) + 0.6595 \, RQ(miR\text{-}215) - 0.4167 \, RQ(miR\text{-}375)$$

wherein IS is the insulitis score and RQ(miR) is the relative quantity of the miR in the serum.

**Table 2:** Association between the relative quantities of the 6 microRNAs tested and degree of insulitis in NOD prediabetic mice from 17 to 43 weeks of age (n=24, multivariable regression, MedCalc software).

| **Multiple regression** | |
|---|---|
| Dependent Y | Score |
| | |
| **Least squares multiple regression** | |
| Method | Backward |
| Enter variable if P< | 0.05 |
| Remove variable if P> | 0.1 |
| | |
| Sample size | 24 |
| Coefficient of determination R$^2$ | 0.3235 |

(continued)

| Least squares multiple regression | | | | | |
|---|---|---|---|---|---|
| Method | Backward | | | | |
| Readjusted | 0.2221 | | | | |
| Multiple correlation coefficient | 0.5688 | | | | |
| Residual standard deviation | 0.7174 | | | | |
| **Regression Equation** | | | | | |
| Independent variables | Coefficient | Std. error | $r_{partial}$ | t | P |
| (Constant) | 0.6535 | | | | |
| miR-7a | 0.2542 | 0.1177 | 0.4350 | 2.160 | 0.0431 |
| miR-215 | 0.6595 | 0.2911 | 0.4519 | 2.266 | 0.0347 |
| miR-375 | -0.4167 | 0.1573 | -0.5097 | -2.649 | 0.0154 |
| Variables not included *in* the model | | | | | |
| Let7e | | | | | |
| miR-29b | | | | | |
| miR-467a | | | | | |
| **Analysis of Variance** | | | | | |
| Source | DF | Sum of Squares | | Mean Square | |
| Regression | 3 | 4.9232 | | 1.6411 | |
| Residual | 20 | 10.2940 | | 0.5147 | |
| | | | | | |
| F-ratio | 3.1884 | | | | |
| Significance level | P=0.0460 | | | | |

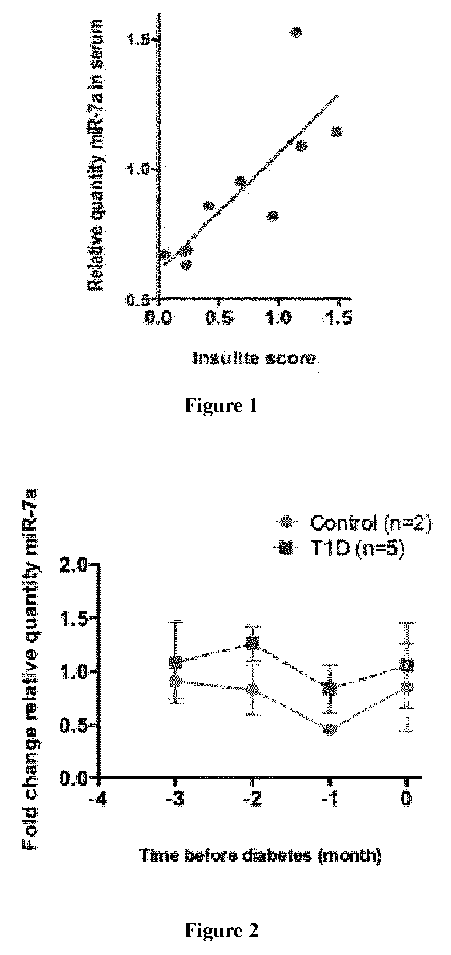

MiR-7a appears to be strongly related to insulitis and type 1 diabetes. The relative quantity of miR-7a in serum was positively correlated with insulitis in NOD mice with early and moderate insulitis score (n=10, p=0.0011 (Spearman), see Figure 1). Among the six miRNAs tested, miR-7a was found to be associated with the onset of diabetes as its relative quantity in the serum of prediabetic NOD mice was much higher than in the serum of diabetic mice at the onset/diagnosis of the disease (n=23, logistic regression, global significance p=0.0105; p=0.0324 for miR-7a, Table 3).

**Table 3:** Association between the relative quantities of the 6 microRNAs tested and onset of type 1 diabetes in NOD mice (comparison of 16 diabetic NOD mice (at the onset of the disease) with 7 prediabetic NOD mice, logistic regression, MedCalc software).

| Logistic Regression | |
|---|---|
| Dependent Y | Statut |
| | |
| Method | Backward |
| Enter variable if P< | 0.05 |
| Remove variable if P> | 0.1 |
| | |
| Sample size | 23 |
| Positive cases [a] | 16 (69.57%) |

(continued)

| Method | Backward |
|---|---|
| Negative cases [b] | 7 (30.43%) |
| [a] Statut = 1 [b] Statut = 0 | |
| | |

**Overall Model Fit**

| Null model -2 Log Likelihood | 28.267 |
|---|---|
| Full model -2 Log Likelihood | 21.724 |
| Chi-squared | 6.544 |
| DF | 1 |
| Significance level | P=0.0105 |

**Coefficients and Standard Errors**

| Variable | Coefficient | Standard Error | P |
|---|---|---|---|
| miR-7a | 4.59387 | 2.14780 | 0.0324 |
| Constant | -3.1319 | | |

| Variables not included in the model |
|---|
| miR-215 |
| miR-375 |
| miR-467a |
| Let7e |
| miR-29b |

**Odds Ratios and 95% Confidence Intervals**

| Variable | Odds ratio | 95% CI |
|---|---|---|
| miR-7a | 98.8760 | 1.4684 to 6657.8523 |

**Hosmer & Lemeshow test**

| Chi-squared | 10.9213 |
|---|---|
| DF | 10 |
| Significance level | P=0.3637 |

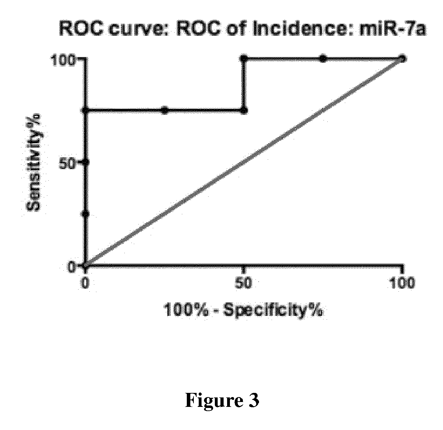

Finally, to estimate the predictive value of the six miRNAs tested, the present Inventors quantified them in the serum of NOD mice from birth until diabetes onset. MiR-7a was detected in higher relative quantity in the serum of NOD mice at least 3 months before the onset of diabetes (n=7, p=0.0476 (Mann-Whitney), see Figure 2). Even if this should be tested using a higher number of animals, the detection of the micro-RNA, miR-7a, during prediabetes could sensitively and specifically predict type 1 diabetes onset (n=7, p=0.083, ROC analyze, Figure 3).

**Claims**

1. *An in vitro* method for predicting future type 1 diabetes development in a subject, the method comprising a step of:

   - determining the level of miR-7a in a biological sample obtained from said subject.

2. The *in vitro* method according to claim 1, wherein the subject is a type 1 prediabetic subject.

3. The *in vitro* method according to claim 1 or claim 2, wherein the biological sample is a blood sample selected from the group consisting of a whole blood sample, a serum sample and a plasma sample or wherein the biological sample comprises lipoproteins, extracellular vesicles, or Argonaute protein complexes extracted from a blood sample taken from said subject.

4. The *in vitro* method according to any one of claims 1 to 3 further comprising a step of:

   - comparing the level of miR-7a measured in the biological sample obtained from the subject to the level of miR-7a measured in a biological sample obtained from at least one healthy subject,

   wherein an increased miR-7a level in the tested subject compared to the healthy subject is indicative of future development of type 1 diabetes in the tested subject.

5. The method according to any one of claims 1 to 4, wherein determining the level of miR-7a is performed using a polymerase chain reaction (PCR) technique.

6. *An in vitro* method for assessing the effectiveness of a therapeutic treatment administered to a type 1 prediabetic subject for preventing future type 1 diabetes development in said prediabetic subject, the method comprising steps of:

(a) determining the level of miR-7a in a biological sample obtained from the type 1 prediabetic subject after administration of the therapeutic treatment; and
(b) comparing the level of miR-7a measured in step (a) to the level of miR-7a measured in a biological sample obtained from the type 1 prediabetic subject before administration of the therapeutic treatment,

wherein a decreased miR-7 level after treatment compared to before treatment is indicative of an effective therapeutic treatment in said type 1 prediabetic subject.

7. A method for screening candidate compounds for their ability to prevent future type 1 diabetes development in prediabetic subjects, said method comprising steps of:

(a) determining the level of miR-7a in a cell after contacting the cell with the candidate compound or determining the level of miR-7a in a biological sample obtained from an animal model after administration of the candidate compound to the animal model; and
(b) comparing the level of miR-7a measured in step (a) to the level of miR-7a measured in a cell before contacting the cell with the candidate compound or to the level of miR-7a measured in a biological sample obtained from the animal model before administering the candidate compound to the animal model,

wherein a decreased miR-7 level after contacting/administration compared to before contacting/administration is indicative of an efficient candidate compound.

8. *An in vitro* method for diagnosing early insulitis in a subject, the method comprising steps of:

(a) determining the level of miR-7a in a biological sample obtained from said subject;
(b) comparing the level of miR-7a measured in step (a) to the level of miR-7a measured in a biological sample obtained from at least one healthy subject,

wherein an increased miR-7a level in the tested subject compared to the healthy subject is indicative of early insulitis in the tested subject.

9. *An in vitro* method for staging or scoring insulitis in a subject, the method comprising steps of:

(a) determining the levels of miR-7a, miR-215 and miR-375 in a biological sample obtained from said subject; and
(b) combining the levels of miR-7a, miR-215 and miR-375 to obtain the stage/score of insulitis in said subject.

10. The *in vitro* method according to claim 9, wherein combining the levels of miR-7a, miR-215 and miR-375 to obtain the stage/score of insulitis in said subject comprises calculating the stage/score of insulitis (IS) as:

$$IS = 0.65 + 0.25\ RQ(miR\text{-}7a) + 0.66\ RQ(miR\text{-}215) - 0.42\ RQ(miR\text{-}375),$$

wherein RQ(miR-7a) is the level of the miR-7a measured in the biological sample, RQ(miR-215) is the level of the miR-215 measured in the biological sample, and RQ(miR-375) is the level of the miR-375 measured in the biological sample.

11. *An in vitro* method for diagnosing insulitis in a subject, the method comprising steps of:

(a) determining the levels of miR-7a, miR-215 and miR-375 in a biological sample obtained from said subject;
(b) combining the levels of miR-7a, miR-215 and miR-375 to obtain a combined value for the subject; and
(c) comparing the combined value obtained in (b) with the combined value obtained by combining the levels of miR-7a, miR-215 and miR-375 measured in a biological sample obtained from at least one healthy subject,

wherein an increased combined value in the tested subject compared to the healthy subject is indicative of insulitis in the tested subject.

12. *An in vitro* method for assessing the effectiveness of a therapeutic treatment administered to a subject to treat insulitis, the method comprising steps of:

(a) determining the levels of miR-7a, miR-215 and miR-375 in a biological sample obtained from said subject after administration of the therapeutic treatment;
(b) combining the levels of miR-7a, miR-215 and miR-375 to obtain a combined value after administration; and
(c) comparing the combined value obtained in (b) with the combined value obtained by combining the levels of miR-7a, miR-215 and miR-375 measured in a biological sample obtained from at said subject before administration of the therapeutic treatment,

wherein a decreased combined value after treatment compared to before treatment is indicative of the effectiveness of the therapeutic treatment in said subject.

13. The *in vitro* method according to claim 11 or claim 12, wherein combining the levels of miR-7a, miR-215 and miR-375 to obtain a combined value comprises calculating the combined value (CV) as:

$$CV = 0.25 \ RQ(miR\text{-}7a) + 0.66 \ RQ(miR\text{-}215) - 0.42 \ RQ(miR\text{-}375),$$

wherein RQ(miR-7a) is the level of the miR-7a measured in the biological sample, RQ(miR-215) is the level of the miR-215 measured in the biological sample, and RQ(miR-375) is the level of the miR-375 measured in the biological sample.

14. A miR-7a primer and/or probe for use in an *in vitro* method of predicting future type 1 diabetes development in a subject, or for use in an *in vitro* method of diagnosing early insulitis.

15. A set of primers and/or probes for use in an *in vitro* method of diagnosing insulitis in a subject, said set comprising at least one miR-7a primer and/or probe, at least one miR-215 primer and/or probe, and at least one miR-375 primer and/or probe.

16. A kit for use in an *in vitro* method of predicting future type 1 diabetes development in a subject, or for use in an *in vitro* method of diagnosing early insulitis, said kit comprising a miR-7a primer and/or probe and instructions to perform a diagnosis according to a method according to any one of claims 1 to 5 and 8.

17. A kit for use in an *in vitro* method of diagnosing or staging insulitis in a subject, said kit comprising at least one miR-7a primer and/or probe, at least one miR-215 primer and/or probe, and at least one miR-375 primer and/or probe, and instructions to perform insulitis staging according to a method according to claim 9 or claim 10, or instructions to perform insulitis diagnosis according to a method according to claim 11 or claim 13.

Figure 1

Figure 2

**Figure 3**

Europäisches
Patentamt

European
Patent Office

Office européen
des brevets

**EUROPEAN SEARCH REPORT**

Application Number

EP 15 20 0884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/134551 A1 (BANYAN BIOMARKERS INC [US]) 11 September 2015 (2015-09-11) * example 3 * | 14-17 | INV. C12Q1/68 |
| X | LOTTE B. NIELSEN ET AL: "Circulating Levels of MicroRNA from Children with Newly Diagnosed Type 1 Diabetes and Healthy Controls: Evidence That miR-25 Associates to Residual Beta-Cell Function and Glycaemic Control during Disease Progression", EXPERIMENTAL DIABETES RESEARCH, vol. 4, no. 9, 1 January 2012 (2012-01-01), pages 1-7, XP055233702, US ISSN: 1687-5214, DOI: 10.3892/or.2011.1530 * pages 3-4 * | 1-7, 14-16 | |
| X | HUANYU XU ET AL: "The circular RNA Cdr1as, via miR-7 and its targets, regulates insulin transcription and secretion in islet cells", SCIENTIFIC REPORTS, vol. 5, 27 July 2015 (2015-07-27), page 12453, XP055259631, DOI: 10.1038/srep12453 * abstract * | 1-7, 14-16 | TECHNICAL FIELDS SEARCHED (IPC) C12Q |
| X | SABIRE ÖZCAN: "Minireview: MicroRNA Function in Pancreatic [beta] Cells", MOLECULAR ENDOCRINOLOGY, vol. 28, no. 12, 1 December 2014 (2014-12-01), pages 1922-1933, XP055259634, US ISSN: 0888-8809, DOI: 10.1210/me.2014-1306 * page 1924 - page 1925 * | 1-7, 14-16 | |

-/--

The present search report has been drawn up for all claims

| Place of search | Date of completion of the search | Examiner |
|---|---|---|
| Munich | 28 June 2016 | Dolce, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 15 20 0884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MATHIEU LATREILLE ET AL: "MicroRNA-7a regulates pancreatic [beta] cell function", JOURNAL OF CLINICAL INVESTIGATION, vol. 124, no. 6, 2 June 2014 (2014-06-02), pages 2722-2735, XP055259638, US ISSN: 0021-9738, DOI: 10.1172/JCI73066 * abstract * | 1-7, 14-16 | |
| X | YIPING MAO ET AL: "MicroRNAs as pharmacological targets in diabetes", PHARMACOLOGICAL RESEARCH, vol. 75, 1 September 2013 (2013-09-01), pages 37-47, XP055091416, ISSN: 1043-6618, DOI: 10.1016/j.phrs.2013.06.005 * page 39 * | 1-7, 14-16 | |
| X | HORSHAM JESSICA L ET AL: "MicroRNA-7: A miRNA with expanding roles in development and disease", INTERNATIONAL JOURNAL OF BIOCHEMISTRY AND CELL BIOLOGY, vol. 69, 10 November 2015 (2015-11-10), pages 215-224, XP029333512, ISSN: 1357-2725, DOI: 10.1016/J.BIOCEL.2015.11.001 * pages 220-221 * | 1-7, 14-16 | TECHNICAL FIELDS SEARCHED (IPC) |
| X | WO 2009/067644 A1 (UNIV MIAMI [US]; PASTORI RICARDO [US]; BRAVO-EGANA VALIA [US]; ROSERO) 28 May 2009 (2009-05-28) * pages 35-38 * | 1-7, 14-16 | |

-/--

The present search report has been drawn up for all claims

| Place of search | Date of completion of the search | Examiner |
|---|---|---|
| Munich | 28 June 2016 | Dolce, Luca |

EPO FORM 1503 03.82 (P04C01)

Europäisches
Patentamt

European
Patent Office

Office européen
des brevets

**EUROPEAN SEARCH REPORT**

Application Number

EP 15 20 0884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | E. ROGGLI ET AL: "Involvement of MicroRNAs in the Cytotoxic Effects Exerted by Proinflammatory Cytokines on Pancreatic -Cells", DIABETES, vol. 59, no. 4, 1 April 2010 (2010-04-01), pages 978-986, XP055284139, US ISSN: 0012-1797, DOI: 10.2337/db09-0881 * abstract * ----- | 8-17 | |

| | | | TECHNICAL FIELDS SEARCHED (IPC) |
|---|---|---|---|

The present search report has been drawn up for all claims

| Place of search | Date of completion of the search | Examiner |
|---|---|---|
| Munich | 28 June 2016 | Dolce, Luca |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 15 20 0884

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 15 20 0884

The Search Division considers that the present European patent application does not comply with the requirements of unity of invention and relates to several inventions or groups of inventions, namely:

1. claims: 1-7(completely); 14-16(partially)

   methods and products to predict type 1 diabetes development, methods for assessing effectiveness of therapeutic treatment of type 1 diabetes, methods to screen compounds to prevent type 1 diabetes based on miR-7
   ---

2. claims: 8-13, 17(completely); 14-16(partially)

   methods and products to diagnose and/or stage insulitis, methods to assess the effectiveness of treatment of insulitis based on miR-7a, alone or in combination with both miR-215 and miR-375
   ---

**EP 3 181 699 A1**

## ANNEX TO THE EUROPEAN SEARCH REPORT
## ON EUROPEAN PATENT APPLICATION NO.

EP 15 20 0884

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2016

| Patent document cited in search report | | Publication date | Patent family member(s) | Publication date |
|---|---|---|---|---|
| WO 2015134551 | A1 | 11-09-2015 | NONE | |
| WO 2009067644 | A1 | 28-05-2009 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009067644 A
- EP 2419536 A
- WO 2012101392 A
- WO 2014022852 A
- EP 2545190 A
- US 5270184 A
- US 5455166 A
- US 4683195 A
- US 4683202 A

- US 4800159 A
- WO 2010085966 A
- US 7575863 B, Chen
- US 6309829 B
- US 5981176 A
- US 5935793 A
- WO 0192579 A
- US 4458066 A
- US 5124246 A

### Non-patent literature cited in the description

- **PATTERSON et al.** *Diabetes Res. Clin. Pract.,* 2014, vol. 103, 161-175
- **TAMAYO et al.** *Diabetes Res. Clin. Pract.,* 2014, vol. 103, 206-217
- **ZIEGLER.** *Nepom, Immunity,* 2010, vol. 32, 468-478
- **WATKINS et al.** *Transl. Res.,* 2014, vol. 164, 110-121
- **SOSENKO et al.** *Diabetes Care,* 2014, vol. 37, 979-984
- **KNIP et al.** *Diabetes Care,* 2010, vol. 33, 1206-1212
- **LANDIN-OLSSON et al.** *Diabetologia,* 1992, vol. 35, 1068-1073
- **LA GASSE et al.** *Diabetes Care,* 2002, vol. 26, 505-511
- **KARJALAINEN.** *Diabetes,* 1990, vol. 39, 1144-1150
- **LATREILLE et al.** *J. Clin. Invest.,* 2014, vol. 124, 2722
- **MU et al.** *PloS One,* 2013, vol. 8, 58622
- **NIELSEN et al.** *Exp. Diabetes Res.,* 2012
- **SALAMA et al.** *PloS One,* 2004, vol. 9, 106153
- **SEBASTIANI et al.** *Diabetes Metab. Res. Rev.,* 2011, vol. 27, 862
- **ERENER et al.** *Endocrinology,* 2013, vol. 154, 603-608
- **J. SAMBROOK et al.** Molecular Cloning: A Laboratory Manual. Cold Spring Harbor Press, 2012
- **F.M. AUSUBEL.** Current Protocols irt Molecular Biology. John Wiley & Sons, 1994
- Molecular Cloning: A Laboratory Manual. Cold Spring Harbor Press, 2012
- **MITCHELL et al.** *Proc. Natl. Acad. Sci. USA,* 2008, vol. 105, 10513-10518

- **J. SAMBROOK et al.** Molecular Cloning: A Laboratory Manual. Cold Spring Harbour Laboratory Press, 1989
- **P. CHOMCZYNSKI ; N. SACCHI.** *Anal. Biochem.,* 1987, vol. 162, 156-159
- **A.R. KIMMEL ; S.L. BERGER.** *Methods Enzymol.,* 1987, vol. 152, 307-316
- **J. SAMBROOK et al.** Molecular Cloning: A Laboratory Martial. Cold Spring Harbour Laboratory Press, 1989
- Short Protocols in Molecular Biology. John Wiley & Sons, 2002
- **WANG et al.** *Nucl. Acids Res.,* 2010, vol. 38, 7248-7259
- **ARROYO et al.** *Proc. Natl. Acad. Sci. USA,* 2011, vol. 108, 5003-5008
- **VICKERS et al.** *Nat. Cell Biol.,* 2011, vol. 13, 423-433
- **WAGNER et al.** *Arterioscler. Thromb. Vase. Biol.,* 2013, vol. 33, 1392-1400
- **NICULESCU et al.** *PLoS One,* 2015, vol. 10, 0140958
- **TURCHINOVICH et al.** *Methods Mol. Biol.,* 2013, vol. 1024, 97-107
- **HAUPTMANN et al.** *Proc. Natl. Acad. Sci. USA,* 2015, vol. 112 (38), 11841-11845
- **FLORES-JASSO et al.** *RNA,* 2013, vol. 19 (2), 271-279
- **ENDERLE et al.** *PLoS One,* 2015, vol. 10 (8), 0136133
- **WELTON et al.** *J. Extracell. Vesicles,* 2015, 27269
- **BÖING et al.** *J. Extracell. Vesicles.,* 2014, vol. 3, 23430
- **GALLART-PALAU et al.** *Sci. Rep.,* 2015, vol. 5, 14664

- **VARALLYAY et al.** *Methods,* 2007, vol. 43, 140-145
- **ACH et al.** *BMC Biotechnol.,* 2008, vol. 8, 69
- **KAUPPINEN et al.** *Methods Mol. Biol.,* 2008, vol. 451, 217-227
- **VARALLYAY et al.** *Nat. Protoc.,* 2008, vol. 3, 190-196
- **LI et al.** *Anal. Bioanal. Chem.,* 2009, vol. 394, 1117-1124
- **LI et al.** *Anal. Chem.,* 2009, vol. 81, 5446-5451
- **KOSCIANSKA et al.** *BMC Mol. Biol.,* 2011, vol. 12, 14
- **ROY et al.** *Lab Chip.,* 2011, vol. 11, 1886-1894
- **BAKER et al.** *Nucl. Acids Res.,* 2012, vol. 40 (2), 13
- **PLANELL-SAQUER et al.** *Clin. Biochem.,* 2013, vol. 46, 869-878
- **HE et al.** *Chem. Commun.,* 2014, vol. 50, 6236-6239
- **TIAN et al.** *Org. Biomol. Chem.,* 2015, vol. 13, 2226-2238
- **URBANEK et al.** *Int. J. Mol. Sci.,* 2015, vol. 16, 13259-13286
- **ALLAWI et al.** *RNA,* 2004, vol. 10, 1153-1161
- **SHI et al.** *BioTechniques,* 2005, vol. 39, 519-525
- **CHEN et al.** *Nucleci Acids Res.,* 2005, vol. 33, 179
- **VARKONYI-GASIC et al.** *Plant Methods,* 2007, vol. 3, 12
- **SCHMITTGEN et al.** *Methods,* 2008, vol. 44, 31-38
- **SIVA et al.** *Mol. Cancer,* 2009, vol. 8, 17
- **YANG et al.** *Plant Biotechnol. J.,* 2009, vol. 7, 621-630
- **WAN et al.** *RNA,* 2010, vol. 16, 1436-1445
- **LU et al.** *J. RNAi Gene Silencing,* 2005, vol. 1, 44-49
- **RAYMOND et al.** *RNA,* 2005, vol. 11, 1737-1744
- **SHARBATI-TEHRANI et al.** *BMC Mol. Biol.,* 2008, vol. 9, 34
- **YAO et al.** *RNA,* 2009, vol. 15, 1787-1794
- **JONSTRUP ET AL.** *Jonstrup,* 2006, vol. 12, 1747-1752
- **DUNCAN et al.** *Anal. Biochem.,* 2006, vol. 359, 268-270
- **ZHOU et al.** *Nucleic Acids Res.,* 2010, vol. 38, 156
- **BALCELLS et al.** *BMC Biotechnology,* 2011, vol. 11, 70
- **CIRERA et al.** *Methods Mol. Biol.,* 2014, vol. 1182, 73-81
- **HUANG et al.** *PLoS One,* 2015, vol. 10, 0120160
- **BUSK.** *BMC Bioinformatics,* 2014
- **CZIMMERER et al.** *PLoS One,* 2013, vol. 8 (1), 55168
- **J. SAMBROOK et al.** Molecular Cloning: A Laboratory Manual. Cold Spring Harbour Laboratory Press, 2012
- PCR Protocols: A Guide to Methods and Applications. Academic Press, 1990
- **P. TIJSSEN.** Hybridization with Nucleic Acid Probes - Laboratory Techniques in Biochemistry and Molecular Biology. Elsevier Science, 1993
- PCR Strategies. Academic Press, 1995
- **S.A. NARANG et al.** *Meth. Enzymol.,* 1979, vol. 68, 90-98
- **E.L. BROWN et al.** *Meth. Enzymol.,* 1979, vol. 68, 109-151
- **E.S. BELOUSOV et al.** *Nucleic Acids Res.,* 1997, vol. 25, 3440-3444
- **D. GUSCHIN et al.** *Anal. Biochem.,* 1997, vol. 250, 203-211
- **M.J. BLOMMERS et al.** *Biochemistry,* 1994, vol. 33, 7886-7896
- **K. FRENKEL et al.** *Free Radic. Biol. Med.,* 1995, vol. 19, 373-380
- **PEARSON et al.** *J. Chrom.,* 1983, vol. 255, 137-149
- **MCFARLAND et al.** *Nucleic Acids Res.,* 1979, vol. 7, 1067-1080
- **MAXAM et al.** *Methods of Enzymology,* 1980, vol. 65, 499-560
- **PIELES et al.** *Nucleic Acids Res.,* 1993, vol. 21, 3191-3196
- **WU et al.** *Anal. Biochem.,* 2001, vol. 290, 347-352
- **MANSFIELD et al.** *Mol. Cell Probes,* 1995, vol. 9, 145-156
- **KRICKA.** *Ann. Clin. Biochem.,* 2002, vol. 39, 114-129
- **VAN GIJLSWIJK et al.** *Expert Rev. Mol. Diagn.,* 2001, vol. 1, 81-91
- **JOOS et al.** *J. Biotechnol.,* 1994, vol. 35, 135-153
- **TEMSAMANI et al.** *Mol. Biotechnol.,* 1996, vol. 5, 223-232
- **HEETEBRIJ et al.** *Cytogenet. Cell. Genet.,* 1999, vol. 87, 47-52
- **LEVENSON et al.** *Methods Enzymol.,* 1990, vol. 184, 577-583
- **PFANNSCHMIDT et al.** *Nucleic Acids Res.,* 1996, vol. 24, 1702-1709
- **NEVES et al.** *Bioconjugate Chem.,* 2000, vol. 11, 51-55
- **SEBESTYEN et al.** *Nat. Biotechnol.,* 1998, vol. 16, 568-576
- The Hanlbook of Fluorescent Probes and Research Products. Molecular Probes, Inc,
- **SERREZE et al.** *J. Immunol.,* 1998, vol. 161, 3913-3918

- **KAINO et al.** *J. Pediatr. Endocrinol. Metab.,* 1998, vol. 11, 267-272
- **MIWAKO ; SHUDO.** *Biol. Pharm. Bull,* 2009, vol. 32, 157-159
- **GOUDY et al.** *J. Immunol.,* 2003, vol. 171, 2270-2278